# EUROPEAN PATENT SPECIFICATION

(11) **EP 1 415 624 B1**
(45) Date of publication and mention of the grant of the patent: **17.05.2006**
(21) Application number: 03025056.7
(22) Date of filing: 30.10.2003
(51) Int. Cl.: A61F 2/44, A61F 2/46, A61F 2/30

(54) **Implant for vertebral replacement and restoration of the normal spinal curvature**
Implantat zum Ersetzen von einem Wirbelkörper und Wiederherstellen der natürlichen Wirbelsäulenkrümmung
Implant pour remplacement vertébral et pour le rétablissement de la courbure normale de la colonne vertébrale

(30) Priority: 30.10.2002 AR 0204138
(43) Date of publication of application: 06.05.2004
(73) Proprietor: Carrasco, Mauricio Rodolfo, City of Buenos Aires (AR)
(72) Inventor: Carrasco, Mauricio Rodolfo, City of Buenos Aires (AR)
(74) Representative: Grünecker, Kinkeldey, Stockmair & Schwanhäusser Anwaltssozietät

(56) References cited:
- WO-A-01/70139
- GB-A- 2 364 643
- US-A- 5 827 328

## Description

This invention in general relates to an implant for the replacement for vertebral bodies and their respective intervertebral disks. Particularly, this invention relates to a vertebral implant for the surgical prosthetic replacement of vertebrae and method of column reconstruction. Said implant is composed of flat frame shaped or trapezoidal, triangulate or rectangular ring-shaped members, emulating the perimeter area of vertebral plates and a telescopic motion column system which may be adapted in its length according to the column defect to be replaced, side supports to be fixed in the vertebral wall and to be constructed as a wedge, slanted bars o tubes to restore the spinal curvature in the replaced area. Furthermore, the implant devices have determined functions and may be exchanged to adjust heights, adjustability, adaptation to the type of vertebra (sacral, lumbar, thoracic or cervical) and the spinal angle to be restored, thus enabling the use of the most convenient implant set once the surgical area has been opened.

### Background

Vertebral injuries which restoration is essential arise from a plurality of causes such as cancerous injuries, fractures caused by vertebral traumatism or vertebral softening such as osteoporosis and vertebral deformity of degenerative origin. Said injuries may harm the normal structure of vertebral bodies and, as a consequence, they may cause column deformities, pain and instability of the supporting structure of the skeleton thus compromising the nervous system, the medulla and its nerves, and causing pain and disabilities and even possible permanent damage.

Among the proposed treatments, the surgical treatment is specifically possible which aims to the neurological damage repair by decompressing the compressed nervous tissue and generating a mechanically stable vertebral segment. In order to achieve said goal, it is necessary that the skeletal defect be replaced and stabilized by means of various osteosynthesis methods to provide for a temporary stability and with the addition of bone grafts, biologically active substances or surgical cement, a longer duration of the altered column segment repair is obtained.

In order to achieve a mechanically efficient bone union, it is necessary to meet several biological and osteosynthesis requirments:
a) use of bone grafts and/or biologically active materials, which must be placed abundantly due to the normal volume loss occurring during the bone callus formation process, and if said goal is not achieved, the structure that has been made may be mechanically weak and thereafter it may be destroyed upon the body weight load and its motions,
b) the most possible contact surface between the vertebral bone ends and the bone grafts in order to favor the formation of the bone callus, since the lack of contact or the contact carried out in small surfaces does not form callus mechanically strong enough to support loads,
c) preferably, those healthy vertebral ends with which said grafts are contacted must have the best blood irrigation in order to favor the rapid incorporation of grafts, and, consequently, it is preferable that said grafts contact the most central part of the vertebra where the so called cancellous bone is with a significant blood irrigation,
d) the osteosynthesis must be fixed in and supported by mechanically resistant bone tissues, said support depending on the stability and durability of the mounts interposed in the spinal column, since it is a structure that must support considerable axial loads and therefore the best areas of the vertebral surfaces for said support are the peripheral areas structurally being the continuation of the vertebral cortical walls,
e) supporting surface of implants with a minimum surface to prevent them from sinking in the bone and causing their subsequent instability in the mount or loss of correction of the spinal column axis upon the collapse of the construction made,
f) said implants must neutralize mechanical loads in the three spatial plans in an equivalent way, otherwise they should associate with various mount systems complementing them,
g) said implants must restore the natural curvature of the spinal column,
h) implants for vertebral replacement, characterized in that they are placed by means of a previous surgical intervention, require the best visual field of the spinal medulla to avoid projecting the graft over the medulla thereby damaging it.

In order to achieve said goals, various implants and their methods of use have been proposed, specifically by means of the intervention of the spinal column in its anterior part and removal of the damaged vertebrae and intervertebral disks. Among the implants known for this purpose, some of them may be mentioned which features are not appropriate for the above-mentioned goals and some examples thereof are provided for hereinbelow.

Implants that are not appropriate to the concept of the above item (a) are disclosed in U.S. Patent No. 5,236,460 by Barber, which relates to a solid device with solid supporting plates which does not enable to arrange the bone in a significant way between the vertebrae; U.S. Patent No. 6,190,201 by Sutcliffe, U.S. Patent No. 6,193,755 B1 by Metz-Stavenhagen and U.S. Patent No. 5,571,192 by Schonhoffer, refer to closed implants that do not enable the adaptation of the graft to the volume or to the necessary closeness to the vertebral ends since said implants must be filled with grafts before being placed in the spinal column defect to be repaired, thus causing the loss of the appropriate contact between the grafts and vertebrae for it is unattached within the implant and without possibilities of arranging it after its implantation since the access to the closed cavity is through small holes.

Other implants that are not appropriate to the concept of the above-stated item (b) are disclosed in U.S. Patent No. 4,932,975 by Main and U.S. Patent No. 5,458,641 by Ramirez Jiménez, both of which have solid or porous platforms in their supporting ends with vertebrae, thus interfering with the close contact of the grafts with the vertebral bone.

Other implants that do not comply with the concepts established in items (c) and (d) are disclosed in U.S. Patent No. 5,336,223 by Rogers U.S. Patent No. 4,657,550 by Daher, U.S. Patent No. 4,554,914 by Kapp, U.S. Patent No. 4,553,273 by Wu, which implant ends rest on the core of the vertebrae, which is the area having the biggest mechanical weakness of the vertebra and the most optimum area for fixation of grafts for bone fixation, but said implants cannot be used for the same reason as previously stated.

Other implants that do not meet the requirements established in the above-mentioned item (e) are disclosed in U.S. Patent No. 5,702,455 by Saggar and U.S. Patent No. 5,989,290 by Harms, both of which support vertebrae by means of laminar walls, thereby enabling the easy sinking and subsequent loosening and the possibility of causing the implant migration.

Furthermore, other implants that do not meet the mechanical requirements of the above-mentioned item "f" are disclosed in U.S. Patent No. 5,443,515 by Averill, U.S. Patent No. 5,290,312 by Kojimoto and Yasui, U.S. Patent No. 5,571,190 by Ulrich and Wolf, U.S. Patent No. 6,176,881 B1 by Schar, Hatebur and Schapfer, which implants only support axial loads and must be placed together with other osteosynthesis systems, such as plates with screws that neutralize those forces to which the vertebral segment is subjected, in other space plans. In pursuit of this goal, U.S. Patent No. 5,916,267 by Tienboon discloses two leaves in the implant ends which are attached at a right angle to the main body of the implant and with a lateral extension to the vertebrae to be fixed with screws, but since said extension is fixed, it does not enable the adaptation to the relative and variable angles of the vertebral bodies in their normal curvature configuration. Likewise, U.S. Patent No. 5,290,312 by Kojimoto and Natsuo and U.S. Patent No. 6,159,211 by Boriani et al do not enable said adaptation to the different vertebral angles in the different spinal column levels. There have been other attempts to stabilize the construction in the three spatial plans, such as the addition of further fixation means to the lateral faces of the vertebrae added to the main element placed between said vertebrae. Said characteristics are exemplified in U.S. Patent No. 5,236,460 by Barber, wherein said fixation means are fixed to the platform of each end, without having the possibility of adaptation to the changes of the vertebral angles, since its extension is at a right angle both in the outlet of the implant body and in its extension and it cannot vary according to the vertebral anatomical changes. In U.S. Patent No. 6,106,557 by Robioneck et al, a lateral plate is added to the main body of the implant. Said plate is fixed in the vertebrae by means of screws, which is similar to one of the variants proposed in U.S. Patents Nos. 5,702,453 and 5,776,198 by Rabbe, wherein a lateral plate is added to the end of the main body of the implant with the same constructive criterion as the above-mentioned patent, since they disclose a variant referring to an extension coming from the platforms of both ends wherein a bar is articulated and wherein said bar ends in a plate having holes for its adaptation to the lateral faces of the vertebrae and its screwing. Another variant of lateral extensions to be fixed to the lateral part of vertebral bodies is disclosed in U.S. Patent No. 6,190,413B1 by Sutcliffe, which has a "L" shaped arm to be screwed to the outer part of the main cylindrical body interposed between the vertebrae and by means of a lateral groove in said arm, where it rests on the vertebra, fixation screws are placed.

Another device is disclosed in U.S. Patent No. 4,289,123 by H.K. Dunn, wherein the vertebrae are separated by means of two parallel bars, which may be adjusted with nuts to fix said separation and said bars are supported by side plates with corresponding holes to accept said bars, fixing said plates to the vertebral walls. Another variant of said device is described in U.S. Patent No. 6,106,527 by Wu and Chen, wherein said bars are not free as the ones in Dunn's Patent but each of them originate in the corresponding side plates and fixation is achieved by means of screws which exert a perpendicular pressure on the bars and it further includes a central plate which lead the bars, reduce the flexing possibility of the bars and is attached to the bars with screws which exert a perpendicular pressure thereon. Both devices having only side plates do not reach the balance of mechanical loads thus forcing the structure of vertebral walls and causing as a consequence a mechanical instability. Furthermore, said devices do not teach any means for the restoration of the spinal curvature.

A usual methodology in the application of vertebral replacements and, specially, in the cervical area, consists in placing a cervical plate in order to fix it to the spinal column with screws. In this way, after having been placed on the solid bone graft, the cervical plate fixes said graft and the vertebrae of the defect ends. This method, which is used in many occasions has several disadvantages, one of which consists in the fact that once the solid bone graft has been placed, the medulla cannot be seen and afterwards when handling the osteosynthesis plate, the graft may be projected into the medulla thereby damaging it without noticing it since it is hidden from view. Among other disadvantages, there is the fix arrangement of the holes in the plates, which turns their adaptation difficult to the places recommended for placing the screws in the vertebral bodies.

Other known systems different from the mentioned traditional osteosynthesis plates have the same difficulty since the require firstly the graft placement and then the immobilization system to be placed on said graft, such as the implants disclosed in U.S. Patent No. 5,620,443 by Gertzbein et al and U.S. Patent No. 6,193,720 B1 by Yuan et al. In said patents, a system of bars outside the spinal column enable the fixation of a graft previously placed between the vertebrae is disclosed.

There have been other attempts to repair the spinal column defect which resort to the placement of closed cages filled in with bone grafts, such as U.S. Patent No. 6,231,610 B1 and Document WO 02/03885 A2 by Michelson. Both of them are useful for being placed between the neighbor vertebrae but they are not appropriate to supplement the lack of several vertebral segments.

Other examples of known implants useful for the replacement for several segments are the above-mentioned U.S. Patents No. 6,159,211 by Boraini, and U.S. Patent No. 5,192,327 by Brantingan. Both of them relate to implants consisting in closed cages, which, upon their placement in the required position and owing to the fact that they lack their own fixation means, need to be supplemented by other osteosynthesis means to keep the construction stability and cannot adapt themselves to the spinal column curvatures.

One of the known ways of obtaining an immediate fixation of mounts with the use of implants for vertebral replacement is the use of surgical cement instead of bone grafts. Said cement is generally used in the fixation of prosthesis to bones. Its more frequent use is for example the fixation of prosthesis for hips, knees and other minor joints. Its use has shown the need of a careful and systematic handling owing to essentially two characteristics of the material such as its exothermal reaction and its appropriate plasticity point. The exothermal reaction is the own characteristic of this kind of plastic material and it is triggered upon joining the liquid portion of the component with the acrylic powder. Said temperature is highly harmful for the nervous tissue, which must be protected. Therefore, the broad visual field of said nervous tissue and a space big enough to handle the acrylic cement is critical in order to avoid irreversible damages.

With regard to the plasticity point of the acrylic mass, it is obtained some minutes after its components have been bonded. The aspect of the appropriate mass to be handled and placed has a consistency similar to that of mastic so that the modeling made by the surgeon's hands enables a modeling appropriate for the cavity to be filled in or as long as necessary to join the vertebral bodies by penetrating its core in cavities previously made. In this way, the spilling of acrylic is avoided thereby preventing it from leaking into other sites where damage may be caused, such as nerves, arteries, or other tissues. Therefore, it is not recommended to use liquid acrylic cement, which in fact is the only form it may be used in hollow, tubular and/or closed implants, where there are small holes through which it may be injected. It is not safe to use it with implants that do not enable a broad visual field of the medulla and nerves, such as implants occupying the central part between the vertebrae and partially hiding the medulla with the risk of failing to notice some cement leakage to its surroundings.

The analysis and study of the prior art enables us make a quite correct classification of the different implants known in the state in the art.

We may group and name as closed system those implants that do not enable a clear visual field of the medulla and nerves and/or do not enable to handle fusion materials such as acrylic cement in the intervertebral area. Then, we may name as open system those implants that do in fact enable them.

Secondly, we may group and name as outer systems those implants that convey mechanical efforts through the outer vertebral faces. Then, we name as inner systems those systems that convey mechanical efforts through the inner area of the vertebral plate.

Examples of closed and inner implant systems are US Patents Nos. 4,932,975; US 5,236,460; US 5,290,312; US 5,571,192; US 5,702,453; US6,106,557; US 6,159,211; US 5,916,267; US 5,360,430; US 5,458,641; US 6,395,030; US 5,192,327; US 5,360,430.

Examples of closed and outer systems are US Patents Nos. 6,193,720 and 6,306,136.

Examples of open and outer systems are US Patents Nos. 4,289,123; US 6,106,527; US 6,136,002; and 5,620,443.

Examples of open and inner systems are US Patent No. 5,062,850 and this invention.

With regard to the implant disclosed in U.S. Patent 5,062,850, it must be pointed out that it lacks the basic properties to meet the requirements stated at the beginning of the background discussion. Said implant is composed of three fix bars and two solid outer plates, which do not enable the fusion between the bone material and the central spongy area of the vertebral plate.

Although the main characteristics of some known implants have been described as a reference, said characteristics not being appropriate for the pursued goal, they share in some aspects said peculiarities. Therefore, there is still a need of an implant for adapting it to mechanical and biological needs of vertebral fixation that facilitate the reconstruction of vertebral defects as well as its mechanical fixation, and the mechanical characteristics that said implant and its mount should have to improve the deficiencies of other implants are the following:
a) having a vertebral supporting base appropriate to maximize stability in the operation of mechanical efforts and enough to avoid any sinking in vertebrae, maximizing the contact area of the fusion material with the spongy tissue of the vertebral plate,
b) providing enough space for placing a considerable volume of bone grafts, or substitutes thereof, and for handling surgical cement or equivalent materials,
c) enabling the direct visual field of the nervous elements during the system mounting to avoid damages caused by implant or instrumental elements;
d) holding vertebrae and stabilizing in the three spatial plans with the own means of the implant;
e) enabling the natural restoration of the spinal curvature in the affected zone,
f) providing enough surface on the vertebral plates to favor the fusion of the bone grafts and the equivalent material with the vertebral body,
g) enabling determination of the approximate separation in situ between the opposite faces of the implant,
h) enabling the exact fixation in situ of the separation of the opposite faces of the implant.

Document GB-A-2364643 discloses a spinal prosthesis comprising a hollow strut terminated by a pair of end caps which are adapted to engage respective vertebrae. A fixing element is connected to each end cap. The end caps may be kidney shaped, or may be formed into a circular, oval, crescent or complex polynomial shape.

Document WO 01/70139A discloses skeletal reconstruction cages including a central body and end caps coupled to the ends of the central body. Each of the central body and the end caps may be provided in different sizes so that cages with varying overall heights, and related angulations, may be created.

Therefore, the object of this invention is an implant for vertebral replacement, which characteristics improve the deficiencies of other implants and enable a better use of the bone grafts for the definite stabilization of the spinal column.

### Summary of the invention

This invention relates to an implant for the vertebral body replacement and its use technique for repairing a defect in the spinal column. It provides for the immediate stabilization to definitely remain incorporated in the body and enables the use of bone grafts or other bioactive substances, or surgical cement, which contribute to the definite mount fixation.

According to the present invention, an implant for vertebral replacement comprises the features defined in claim 1.

One of the objectives of this invention consists in providing for an implant with components having standard measures, adaptable in its length to the needs of each case, and with a robust construction for the spinal column stabilization in all the plans of physiological load of the spinal column.

Another objective of this invention consists in providing for an open implant, which enables the use of a considerable bone volume between the columns and since their ends are frames that leave a vertebral surface exposed in each end, said open implant enables an increased contact between vertebrae and grafts, thereby favoring the fixation thereof and the formation of a robust bone callus.

Another objective of this invention relates to the generation of a strong support for the axial load of the spinal column provided by a set of supporting columns arranged at the angles of a supporting frame in the periphery of the vertebral bodies, which is the structure having the best mechanical resistance, leaving the core of the vertebrae free, and being said core the most optimum part for bone fixation, to contact the bone graft mass.

Another objective of this implant consists in the mechanical stabilization in several spatial plans by means of fixation to the upper and bottom vertebral bodies by means of screws arranged in different spatial plans.

Among other benefits, during a surgical intervention, there is a benefit that consists in that the open form of the implant enables the permanent visual field of the medulla during all of the handlings of its mount and the placement of the grafts, thus avoiding any unnoticed damage of the nervous system.

### Brief descriptionof the drawings

- Fig. A:: General view of implant for the lumbar area including two bars
- Fig. B:: View of the jig for angular correction
- Fig. C:: View of bars at different lengths, and pieces with horizontal slipping and inclined pieces
- Fig. D:: General view of the implant for the cervical area including two bars
- Fig. E:: View of direction for screws and alternative cervical shape

Fig. 1: Explosion view of implant I provided with appropriate components for the replacement for lumbar vertebrae or thoracic-lumbar vertebrae.
Fig. 2. Explosion view of implant II, provided with components for the replacement for lumbar vertebrae and the sacral bone.
Fig. 3: Oblique view of implant I placed between two lumbar vertebrae and the screws adjusting said vertebrae.
Fig. 4: Oblique view of implant II placed between a lumbar vertebra and the sacral bone..
Fig. 5: Side view of implant I placed between two lumbar vertebrae with vertebral fixation screws placed.
Fig. 6: Side view of implant II placed between one lumber vertebrae and the sacral bone, with vertebral fixation and telescopic system fixation screws
Fig. 7 A: Front view of implant I placed between two lumbar vertebrae with vertebral fixation screws and telescopic system fixation screws.
Fig. 7 B: View of piece 1, top view of the implant with a cut line AA.
Fig. 7 C: View of pieces 1 and 2 assembled and placed under the lumbar vertebra, wherein the cut AA of piece 1 and the adaptation of the vertebra by a removal from the vertebral body are shown
Fig. 8: Front view of implant II placed between a lumbar vertebra and the sacral bone with its fixation screws, specially showing the ones that are fixed to the sacral bone.
Fig. 9A: Lateral view of piece 2 where the telescopic system tubes and a cut line are seen.
Fig. 9B: Lateral view of piece 1 by the cut line AA, where the direction of the threaded inner tubes for the placement of screws blocking the telescopic system.
Fig. 10 A: General view of the assembled implant I and the approached vertebral supporting platforms, prepared to be placed in the vertebrae.
Fig. 10 B: General view of the assembled implant I and with the vertebral supporting platforms separated between the vertebrae and the telescopic mechanism blocked by the corresponding screws.
Fig. 11 A: Explosion view of implant III provided with appropriate components for the replacement between thoracic vertebrae.
Fig. 11 B: Lateral view of an implant placed with vertical extensions for its lateral fixation.
Fig. 12: Scheme of a predetermination system between bars and indented tubes.
Fig. 13 A: Top view of lumbar, thoracic and cervical vertebral plates, and indication of the cortical tissue.
Fig. 13 B: Top view of lumbar, thoracic and cervical vertebral plates and indication of the shapes provided to the vertebral supporting pieces.
Fig. 14 A: Explosion view of implant IV provided with appropriate components for the replacement between cervical vertebrae.
Fig. 15: Oblique view of a mounted cervical implant.
Fig. 16: Lateral view of an implant where the angular correction achieved by wedged pieces is seen.
Fig. 17: Oblique view of an implant supported by a nipper designed for the placement thereof.
Fig. 18: Lateral view of an implant held by the nippers designed for its placement and exhibited between two vertebrae.
Fig. 19 A: Oblique rear view of an adaptation of the cervical implant for its placement in the axis bone.
Fig. 19 B: Oblique anterior view of an adaptation of the cervical implant for its placement in the axis bone.
Fig. 19 C: Oblique rear view of an adaptation of the cervical implant placed in the axis bone.
Fig. 20: Explosion view of a mount alternative system of an implant for vertebral replacement.
Fig. 21: Oblique view of an implant held by holding nippers.
Fig. 22: Oblique view of an implant with a system of stops for the fixation screws to the vertebral bodies.

### Detailed description of the preferred embodiments of the invention

The following description is the preferred embodiment to carry out the invention and it aims to illustrate the general principles of its use and it must not be considered as limiting the possibilities thereof, its claims defining its scope.

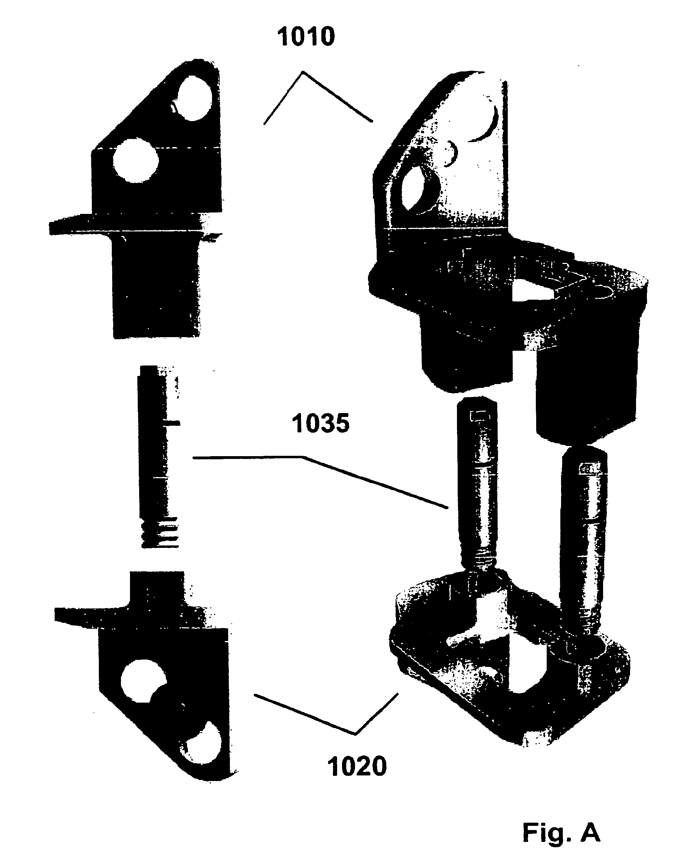

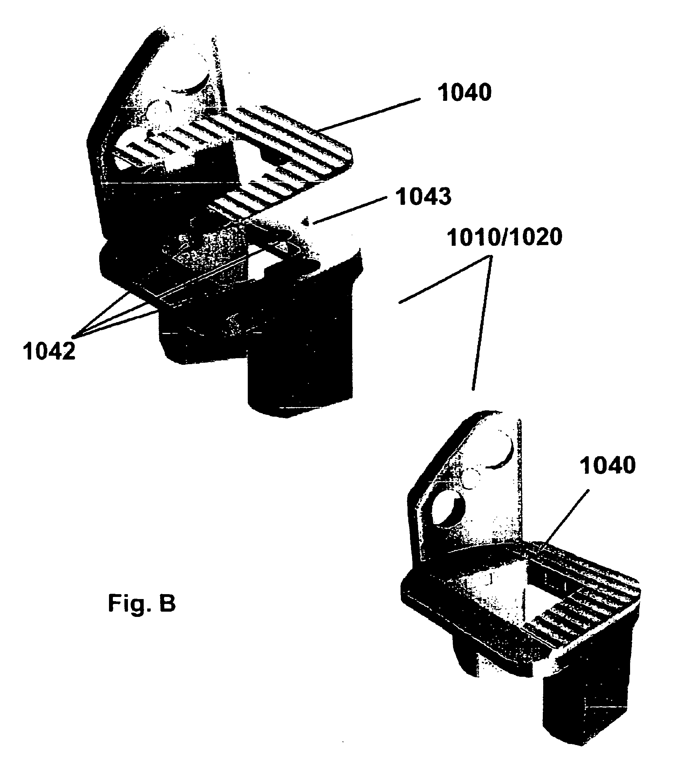

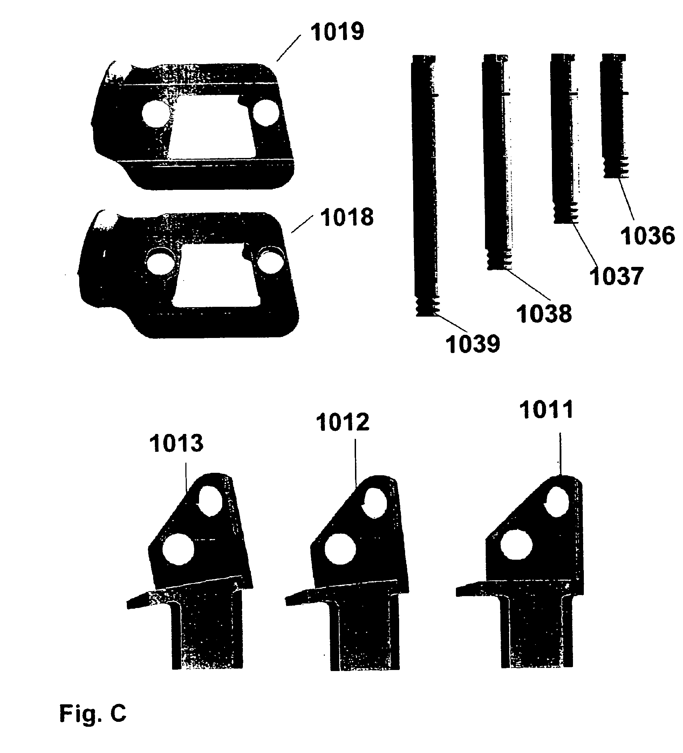

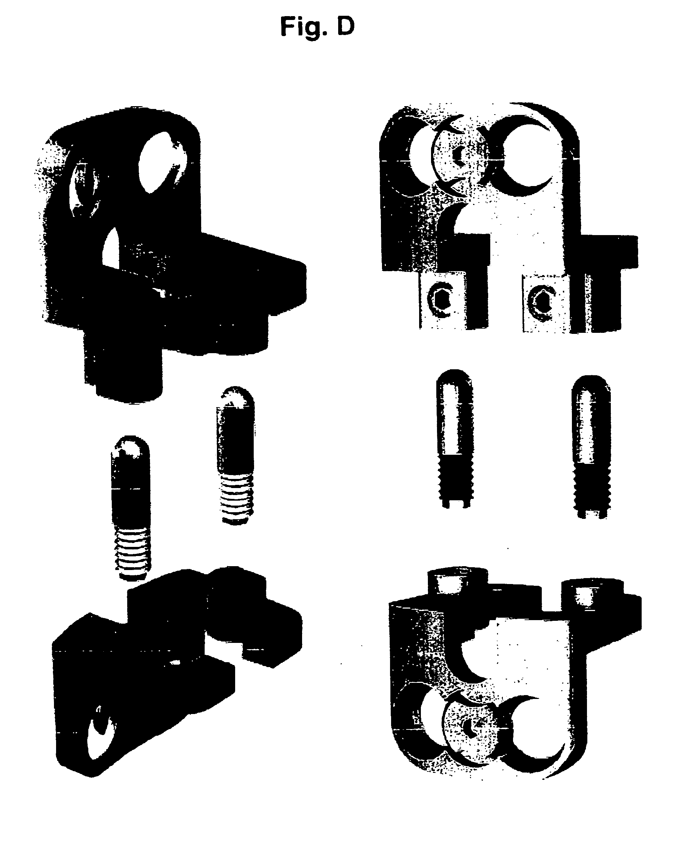

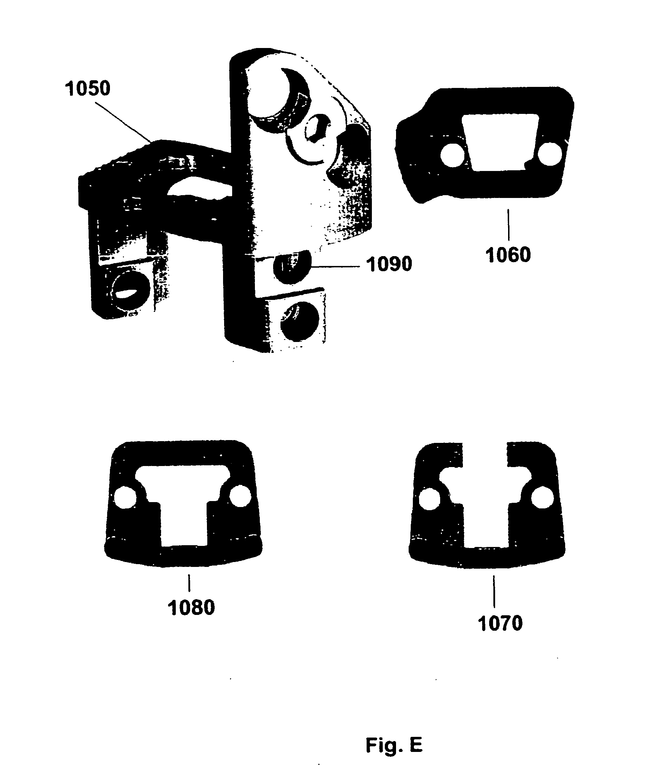

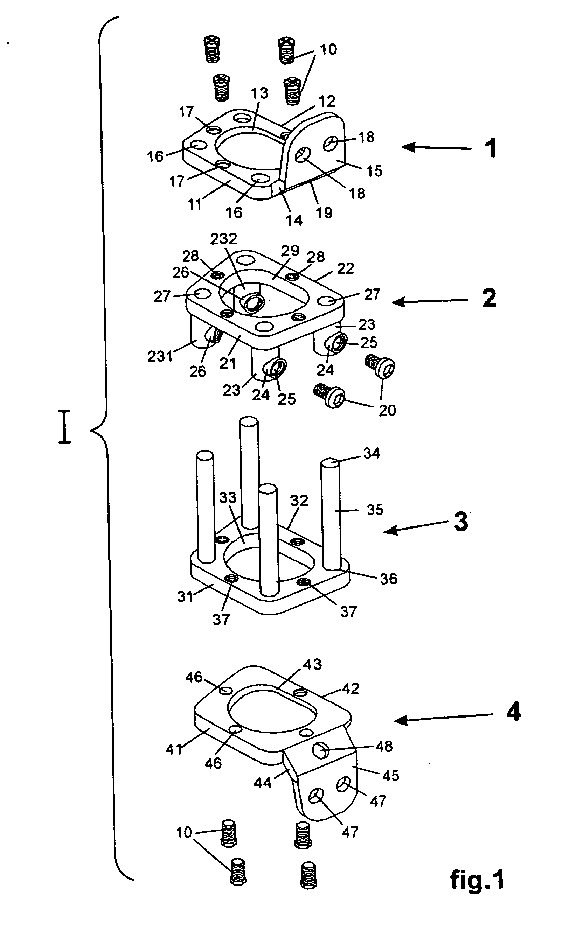

In a preferred embodiment, in Fig. 1, an explosion view of implant I is shown in an assembly embodiment to be placed between two lumbar vertebrae.

The parts thereof are the following:
Piece 1 is a top vertebral supporting frame composed of a trapezoidal shaped horizontal wedged member corresponding to the perimeter of the vertebral plate of the lumbar vertebrae and a vertical extension for the adjustment thereof to the lateral vertebral face.
Piece 2 is a telescopic adjusting frame, with a plan horizontal member having a trapezoidal shape suitable for its adjustment to piece 1 and four vertical extensions consisting in tubes perpendicular to the horizontal member which will receive the bars of piece 3.
Piece 3 is a vertebral separation frame, with a plan horizontal frame having a trapezoidal shape suitable for its adjustment to piece 4 and four vertical bars consisting in bars perpendicular to the horizontal member which will introduce themselves in the tubes of piece 4.
Piece 4 is a bottom vertebral supporting frame, composed of a trapezoidal shaped horizontal wedged member corresponding to the perimeter of the vertebral plate of the lumbar vertebrae and a vertical extension for its adjustment to the lateral vertebral face.

Piece 1 comprises a trapezoidal ring shaped flat member with an outer anterior and short side 11 and an outer rear opposite and longer side 12, parallel between each other, and an outer right side and a vertical left extension 14 that joins the anterior and rear non parallel sides thus closing the frame. The frame vertices are curved. Its hollow interior is determined by the interior perimeter 13.

The height of the section corresponding to the anterior side 11 is higher than the height of the section corresponding to the rear side 12, thus forming a kind of wedge that enables restoration of the preexisting angle between the vertebrae.

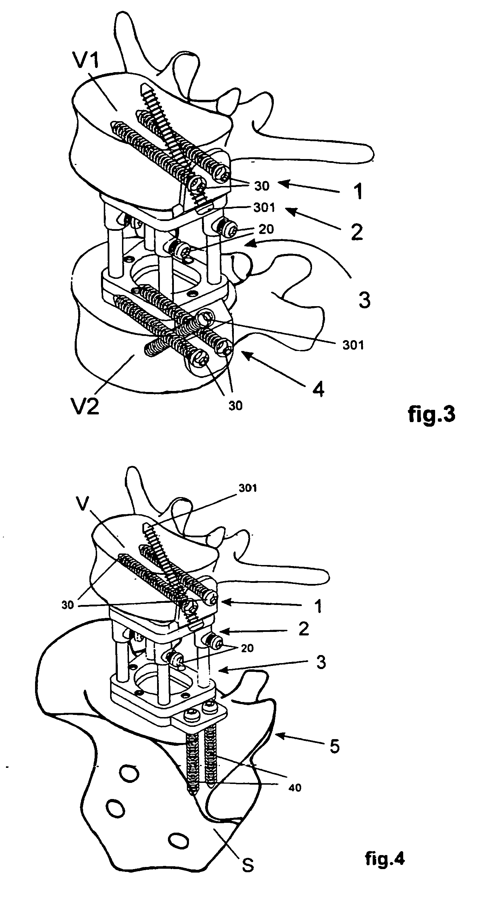

The lateral extension 14 has a hole 19 and continues on the laminar surface 15, which has two holes 18. Said two holes 18 and the hole 19 have a size for the passage of screws 30 and 301 shown in Figs. 3, 4, 5 and 6. The arrangement of said holes enable the spatial fixation of the piece upon providing it with three fixation plans defined by the three screws 30 and 301. Piece 1 has four holes 17 perpendicular to their thickness for the passage of four screws 10, and four holes 16, each of them in each angle, having a size for the passage of bars 35.

Piece 2 comprises a trapezoidal ring shaped flat member with an outer anterior short side 21 and an outer rear opposite and longer side 22, both being parallel sides, and a right side and a left side that join at the anterior and rear side thus closing the frame which corresponds with piece 1. The vertices of the frame are curved. The hollow interior of the frame is determined by the inner perimeter 29.

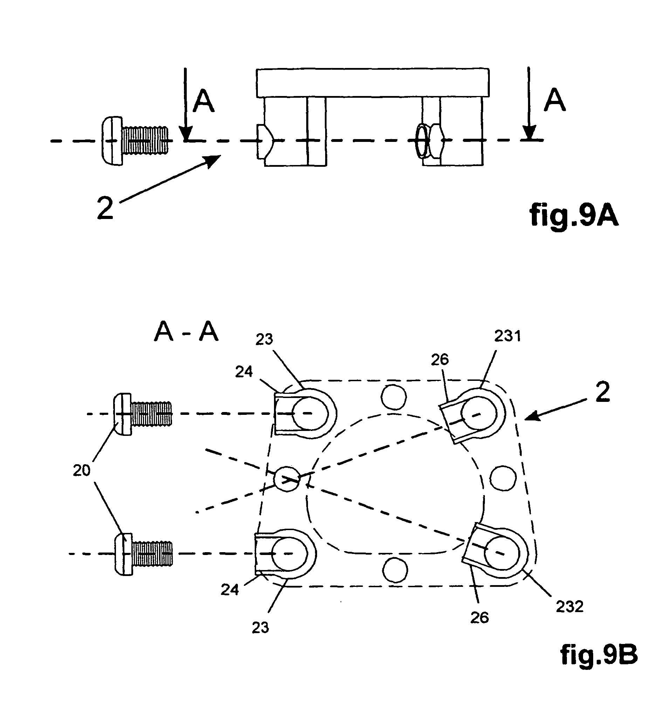

It has two attached tubes 231 and 232 and two tubes 23, all of them with attached tubes 26 and 24 with an inner thread 25 where four screws 20 are threaded. The attached tubes are parallel to each other and convergent 26 and oriented to a middle point between both tubes 23 to enable the introduction of a screwdriver between both tubes 23, to adjust the screws 20 in the tubes 26 as shown in Fig. 9 B.

The frame 2 is adapted to frame 1 as indicated in the explosion view of Fig. 1, coinciding the edges 11 and 12 with the edges 21 and 22 respectively, and by means of screws 10 which penetrate into the smooth holes 17 and are threaded in the threaded holes 28, both pieces 1 and 2 are fixed and in this way the four holes 16 of frame 1 are in continuity with the holes of frame 2 for the passage of bars 35, upon introducing their ends 34 into the tubes 232, 232 and two tubes 23.

Piece 3 comprises a trapezoidal ring shaped flat member with an outer anterior short side 31 and an outer rear opposite and longer side 32, both being parallel sides, and a right side and a left side that join at the anterior and rear sides thus closing the frame which corresponds with piece 4. The vertices of the frame are curved. The hollow interior of the frame is determined by the inner perimeter 33.

Piece 3 includes four threaded holes for screws 10, and in each angle 36 a bar 35 is fixed with free ends 34.

Piece 4 comprises a trapezoidal ring shaped flat member with an outer anterior short side 41 and an outer rear opposite and longer side 42, both being parallel sides, and an outer right side and a vertical left extension 44 and 45 that join at the anterior and rear sides thus closing the frame. The vertices of the frame are curved. The hollow interior of the frame is determined by the inner perimeter 43

The height of the section corresponding to the anterior side 41 is higher than the height corresponding to the rear side 42, thus forming a kind of wedge that enables the restoration of the preexisting angle between the vertebrae.

The lateral extension 44 continues in extension 45. Said extension 44 has a hole 48, and extension 45 has two holes 47, said holes 48 and 47 being for the passage of screws 30 and 301, as may be seen in Figs. 3, 4, 5 and 6 and they achieve the same spatial fixation as described for piece 1.

The assembly of pieces 3 and 4 is carried out as shown in Fig. 1, causing the anterior parts 31 and 41 to coincide with each other, and the rear parts 42 and 32 to coincide with each other. By means of screws 10 that penetrate through the holes 46, the threaded holes 37 are adjusted and pieces 3 and 4 are thereby formed.

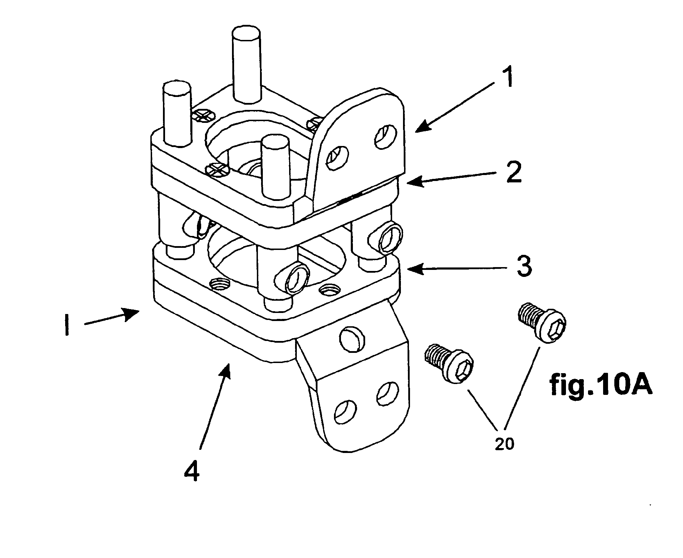

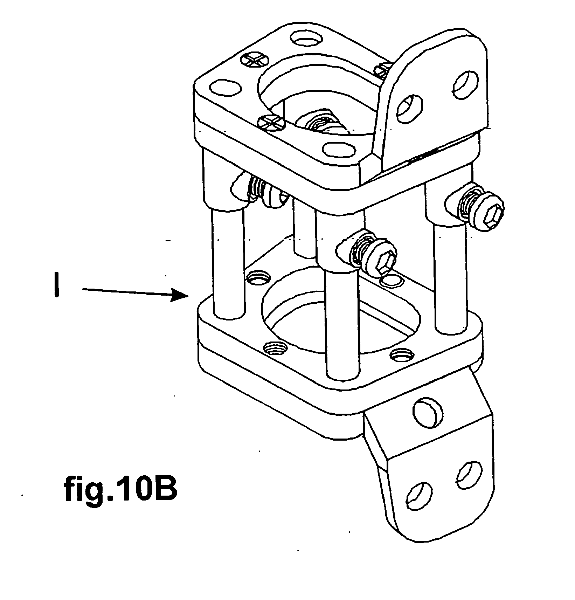

The assembly of the set of pieces of the upper end 1-2 with the set of pieces of the bottom end 3-4 is carried out as shown in Fig. 1, penetrating the bar ends 35 through the tube ends 231, 232 and the two tubes 23, so that the anterior parts 11, 21, 31 and 41 coincide with each other and the rear parts 11, 22, 32 and 42 also coincide with each other, thus forming a preferred embodiment of the formation of implant 1, and comprising a telescopic motion device, enabling the approach or the separation of the ends of implant I as shown in Figs. 10A and 10 B respectively.

As an illustration of this preferred embodiment, Fig. 3 is an oblique image with implant I placed between two vertebrae V1 and V2; the assembled pieces 1, 2, 3 and 4 form implant I of Fig. 10 B, and said implant is placed with the sutiable construction way for two lumbar vertebrae V and V2. Screws 30 and 301 are seen to fix vertebrae V1 and V2 and they are placed in ends 1 and 4. The screws 20 placed for blocking the telescopic system are also illustrated.

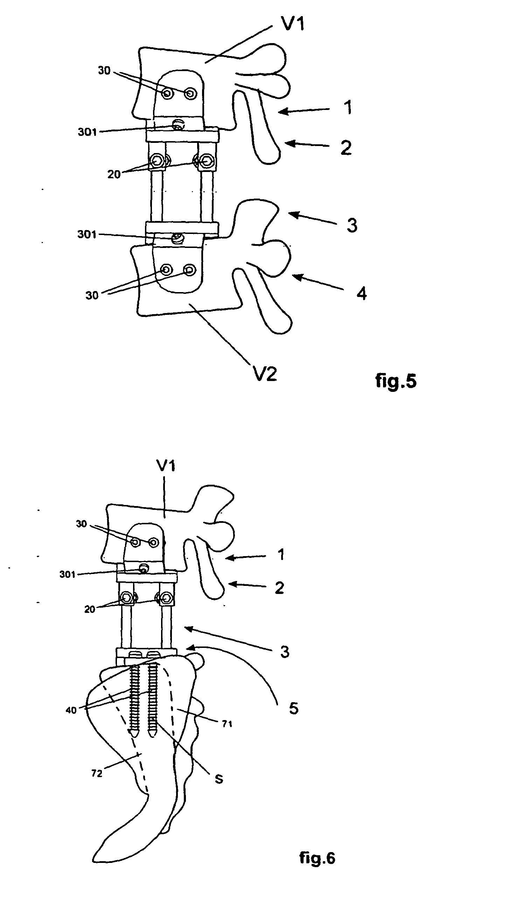

In Figure 5, in a side view, implant I of Figure 10 B is shown with its constituent parts 1, 2, 3 and 4, placed between two lumbar vertebrae V1 and V2, with vertebral fixation screws 30 and 301, and with screws 20 for the telescopic system fixation.

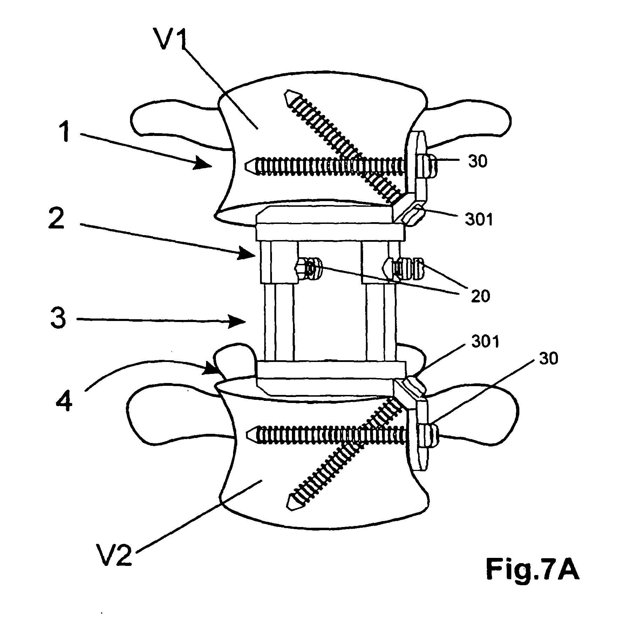

And in Figure 7 A, in a front view, an image of implant I is shown with its assembled parts 1, 2, 3 and 4, placed between two lumbar vertebrae V and V2. Screws 30 and 301 placed in vertebrae V1 and V2 and the screws 20 for the telescopic system fixation are also shown.

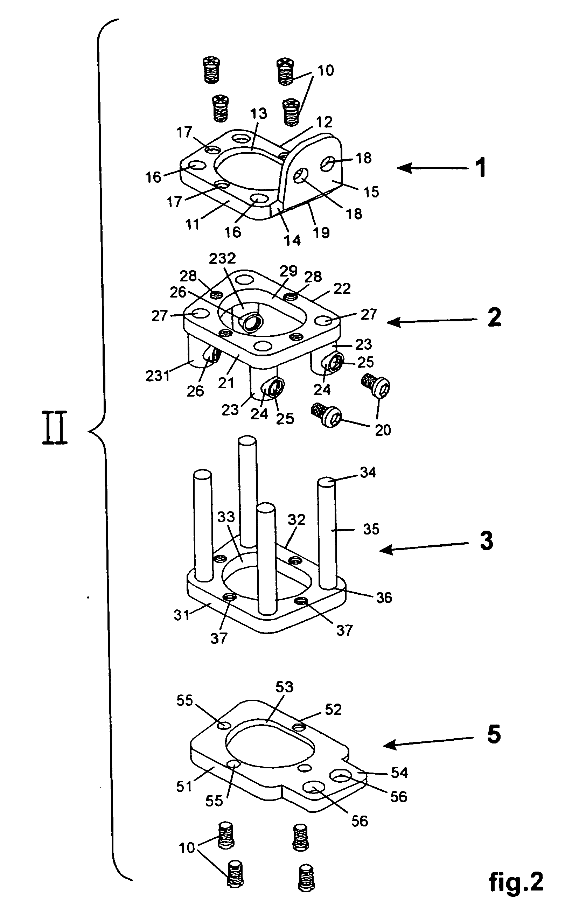

In another preferred embodiment, in Fig. 2, implant II is represented in a suitable way to be placed between a lumbar vertebra and the sacral bone. The component pieces 1, 2 and 3 of the implant have the same individual description as the above-provided description for the implant between lumbar vertebrae.

Piece 5 in Figure 2, which is suitable for the sacral bone, has an anterior part 51 higher than the rear part 52, and a curved inner perimeter 53, which acquires a trapezoidal shape identical to frame 3, but with a lateral wedged aspect. There are four smooth holes 55 in its thickness for the passage of screws 10. In horizontal extension of its surface towards one of the sides 54, there are two holes 56 for the passage of screws 40, as seen in Figs. 4, 6 and 8.

The assembly of implant II is carried out by firstly forming the set of pieces 1 and 2 as hereinabove described, and the set of pieces 3 and 5 causing its anterior parts 31 and 51 to coincide with each other and the rear parts 32 and 52 to also coincide with each other in order to afterwards fix both parts 3 and 5 by means of the screws 10 which penetrate through the holes 55 and are threaded in holes 37.

In another illustration of this preferred embodiment, in Fig. 4, in an oblique view, the assembly of implant of Fig. 1 is shown with its constituent parts 1, 2, 3 and 5, to be placed between a lumbar vertebra from the top, and the sacral bone from the bottom, where the set of pieces of the upper end 1-2 is assembled with the bottom end 3-5. In the bottom supporting piece, the arrangement of fixation screws 40 penetrating the sacral bone may be seen. Screws 30 and 301 are shown fixing vertebra V and screws 20 are shown blocking the telescopic system of the sacral-lumbar implant.

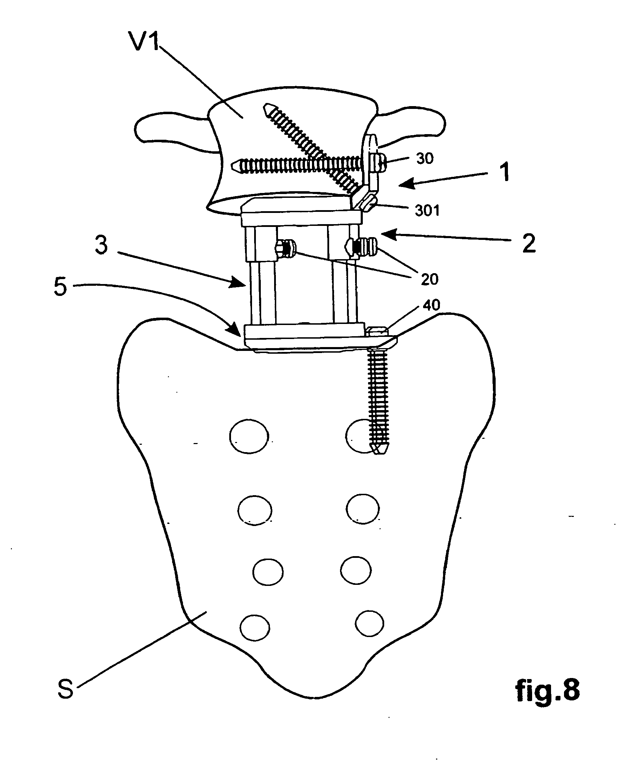

Another aspect of said mount is shown in Figure 6, a side view of the scaral-lumbar implant of the assembly with its constituent parts 1, 2, 3 and 5, placed between the lumbar vertebra V and the sacral bone S. The lumbar vertebral fixation screws 30 and 301 are shown placed and screws 40 for fixation to the sacral bone are also shown. In Fig. 8, there is a front view of the assembly of implant II with its constituent parts 1, 2, 3 and 5, placed between a lumbar vertebra VI and the sacral bone S. The lumbar vertebral fixation screws 30 and 301 and screws 40 for fixation to the sacral bone S may be seen. Likewise, the telescopic system fixation screws 20 may be seen.

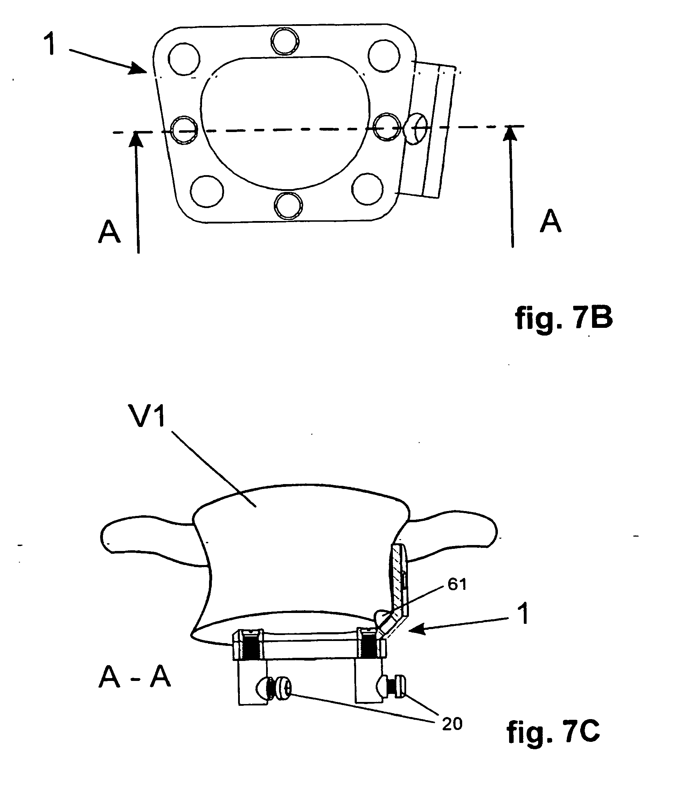

A detail in the placement of the implant and its adaptation to the vertebrae of pieces 1 and 4 of the ends, is the need of removing a small portion of the vertebra 61 in its flange as seen in Fig 7C for the suitable support of the lateral extension 14 and for the extension 44 with regard to the bottom vertebra.

Both of said preferred construction embodiments forming the sacral-lumbar and lumbar implants correspond to the need of preparing them to adapt the different vertebrae and in both ways, they comprise a telescopic system, which for constructive purposes, the load forces supporting the spinal column in a low level of the lumbar column were considered and for which the bars 35 had a diameter size of 4 mm, the frames of pieces 2 and 3 also have a minimum thickness of 4 mm, thus obtaining an enough strength of the structure when built with stainless steel or titanium alloys. Likewise, the additional ends 1, 4 and 5 of the implant add certain thickness to the bases of implants I and II and the pieces of said ends are joined by four screws 10, and without damaging the base strength, a system for the reciprocal insertion of the pieces may be added in its design thereby increasing its grabbing capability. The fixation of the formed telescopic system, the bars and the tubes are individually blocked to provide for a solid grabbing mechanism, with a support of the spinal column axial forces in four points, and a fixation to the vertebrae of at least two screws each.

Further to the sacral and lumbar area, it is also an object of this invention to provide the thoracic as well as the cervical area with the same replacement system, for which the corresponding adaptations are described hereinbelow.

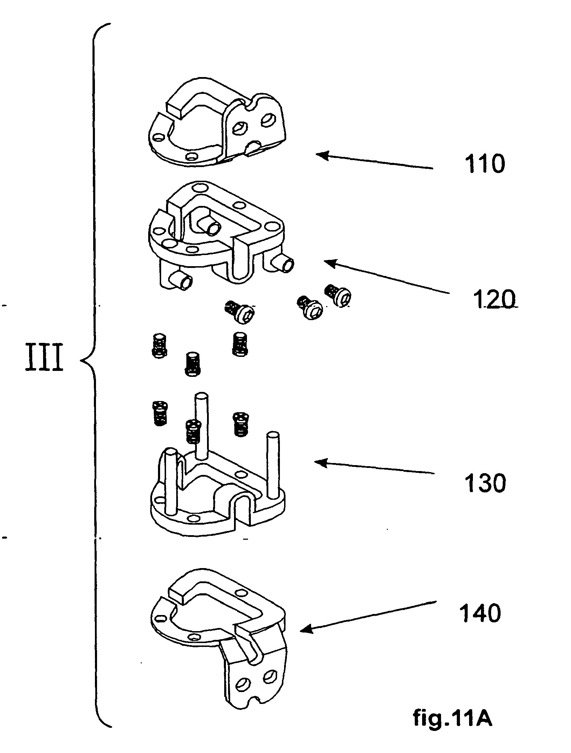

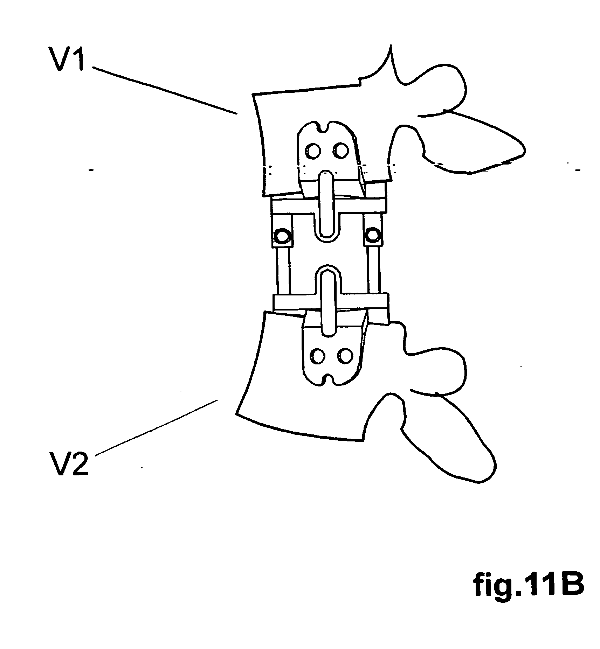

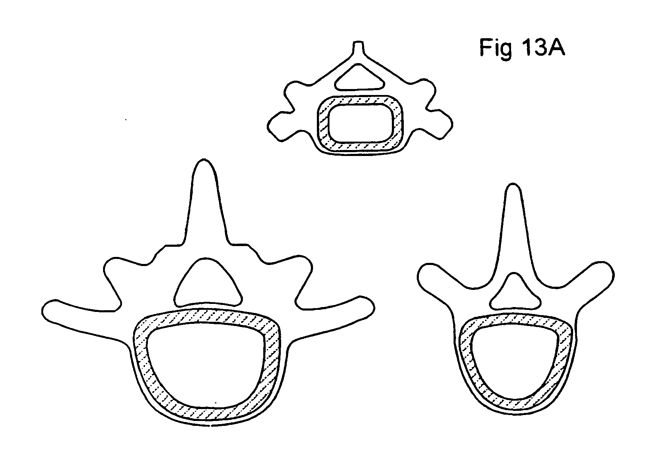

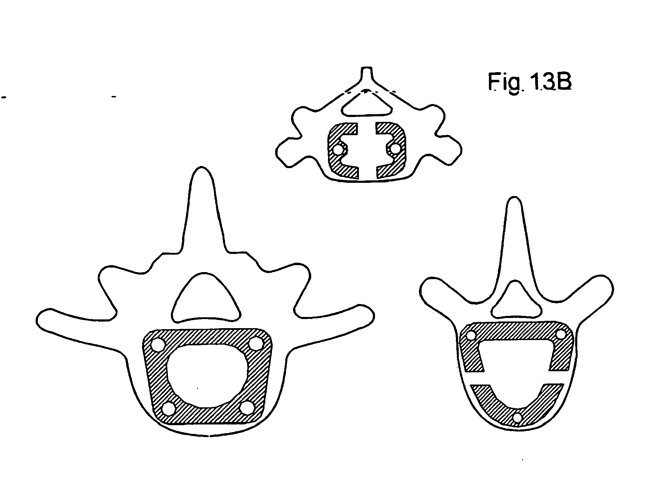

Particularly, and following the stability principle, the pieces comprising a vertebral replacement for the thoracic area are comprised with a triangulate shaped flat member as shown in Figs. 11 A, 13A and 13B, corresponding with the perimetrical shape of the thoracic vertebral plates and due to the triangulate configuration and in relation with the minor magnitude of the loads of said area and, as shown in Fig. 11 A, 3 bars will be arranged in piece 130 with their corresponding 3 tubes in piece 120. Said bars and tubes will be located at the vertices of the triangulate configuration. In the same way, a threaded hole per side will be arranged for joining pieces 1 and 2, and 3 and 4. With regard to the wedge in pieces 110 and 140, it will have a side of a higher height and an opposite vertex of a lower height or vice versa.

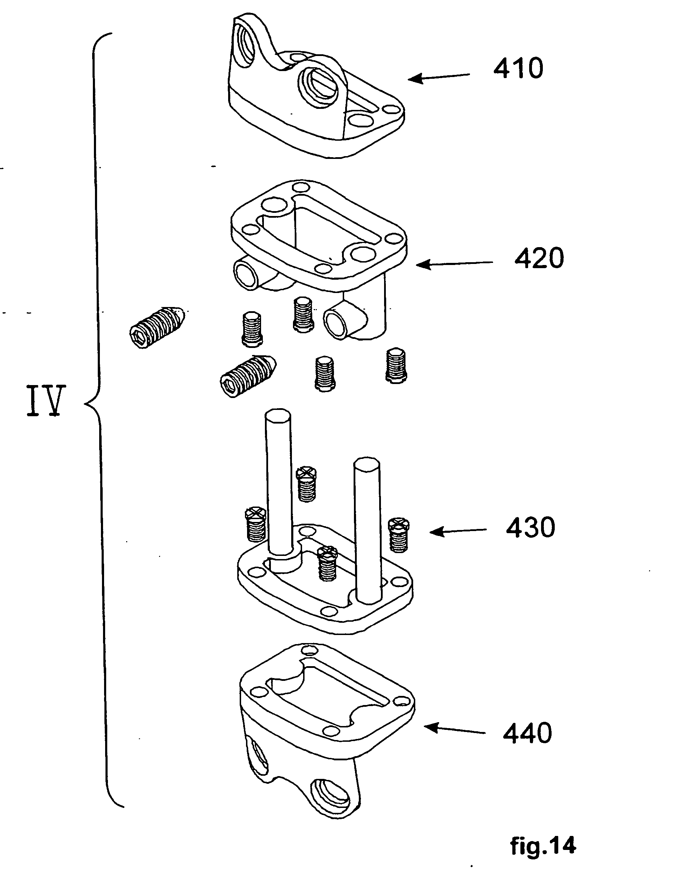

With regard to the pieces that form a vertical replacement for the cervical area, they will be comprised by a rectangular ring shaped flat member with an extension in the center of each smaller side as shown in Figs. 14, 13A and 13B, which supports the bars and tubes, corresponding with the perimetrical form of the cervical vertebral plates, and owing to the rectangular configuration and in relation with the smaller load magnitude in said area, 2 bars will be arranged in piece 430 with their corresponding 2 tubes in piece 420. In the same way, a threaded hole will be provided for each bigger side for joining pieces 410 and 420 and pieces 430 and 440. The smaller sides with their extensions emulate an E shape.

The shapes given to the pieces according to the cortical surface in the vertebral plates are shown in Figures 13 A and 13 B.

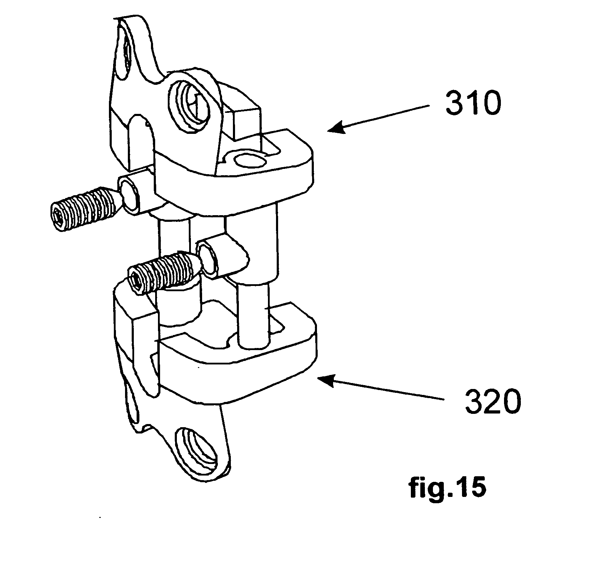

Particularly, for the replacement in the cervical area and owing to space requirements and the reduced mechanical loads, another preferred embodiment of this invention consists in using two pieces instead of four of them, wherein a piece A and a piece B are formed. Additionally, a series of incuts are indicated for a method of placement of the implant. Said mounted device is illustrated in Fig. 15.

Piece 310 is an upper vertebral supporting frame composed of a horizontal wedged member having a rectangular ring shape with an extension in the center of each smaller side serving as a support for the telescopic adjusting tubes, and a vertical extension having two central incuts, a bottom and a top incut, for using accessory instruments which may facilitate the preciseness in the placement thereof.

Piece 320 is a bottom vertebral supporting frame composed of a horizontal wedged member having a rectangular ring shape with an extension in the center of each smaller side serving as support for the vertebral separation bars, and a vertical extension having two central incuts, a bottom incut and a top incut, for using accessory instruments that facilitate preciseness in the placement thereof.

The horizontal member of the vertebral supporting frame, both top and bottom ones, may be interrupted in its continuation in the center of its opposite bigger side next to the place where the vertical extension originates, and said space is part of the incut set of the placement method by means of suitable instruments.

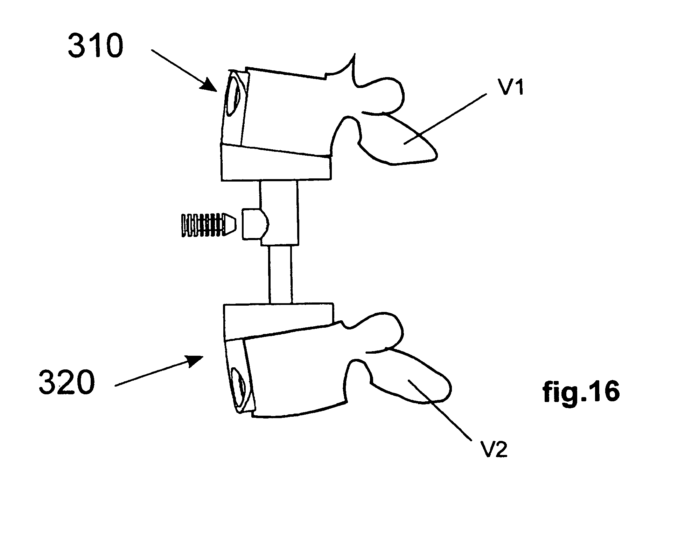

Both pieces have their corresponding holes for the insertion of screws, which will fix said pieces to the vertebral bodies as described in the previous embodiments. Likewise, the bars are blocked by means of the system described in the previous embodiments. The wedge effect on the horizontal members of the pieces are illustrated in the side view of an implant placed according to Figure 16.

The two-piece system may also be suitable for thoracic or lumbar replacements, as long as material sufficiently resistant to the mechanical efforts to be supported is used. Under the same principle, the amount of bars used may be reduced.

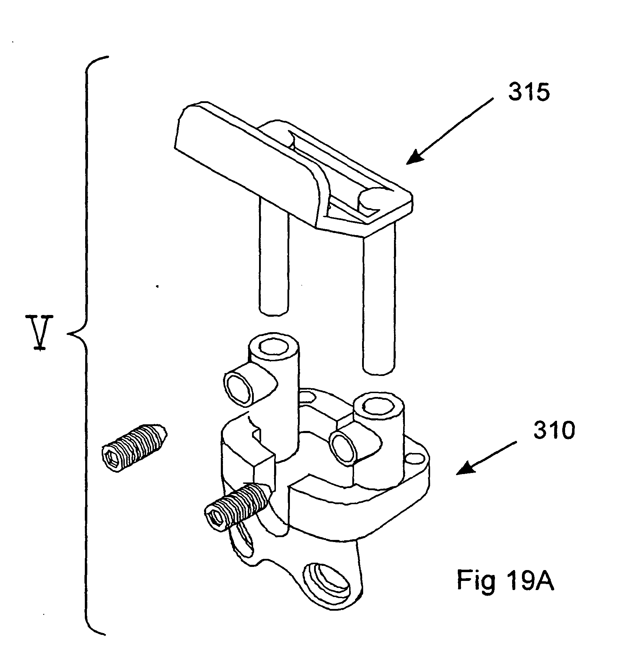

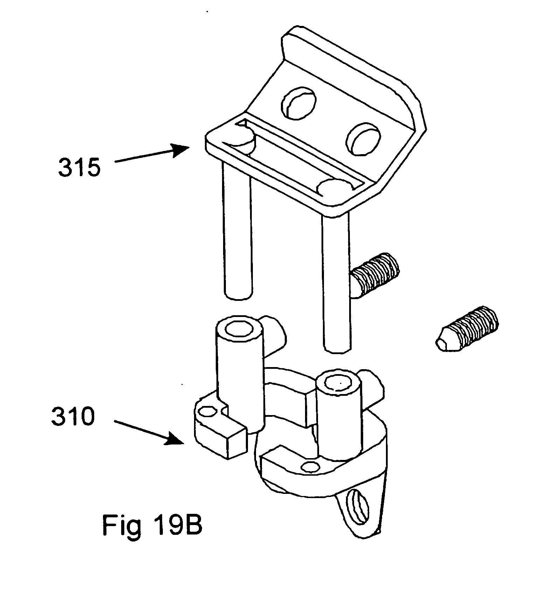

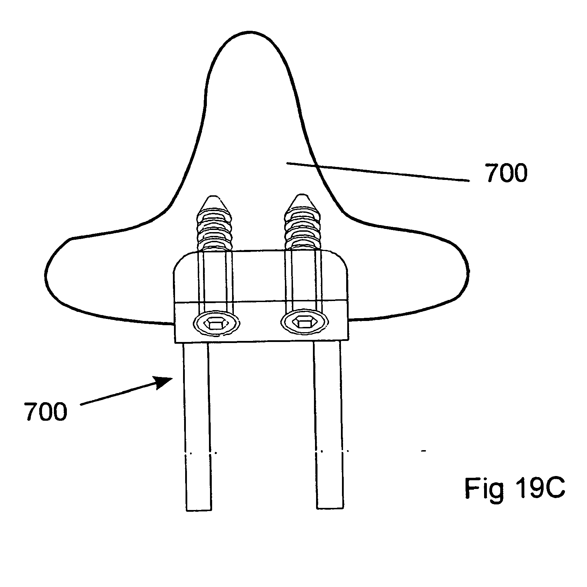

With regard to the cervical area, and in the same way as an adaptation becomes necessary for the bottom end of the spinal column to the sacral bone, the same happens with the axis bone. Figures 19 A and 19 B illustrate a variant composed of a piece 315 which may include bars or tubes as perpendicular projections, and which upper member consists of three flat members, one of them is horizontal and supports the bars, the second one is joined to the first one forming a 135 and 170° angle; and a third member following the previous one forming a 90° angle approximately. In the second member, there are two holes for the passage of screws to be introduced and which will adjust the axis bone, which owing to its structure cannot be correctly fixed at a vertical angle. Figure 19 C illustrates the adaptation placed in the axis bone. Figures 19 A and 19 B are examples of the inversion of bars and tubes, which may be indistinctly built in all the models with any of said configurations.

After having described the characteristics of each piece, the possibility of having variants of each one with predetermined angles, as well as predetermined tube and bar lengths becomes apparent in order to obtain, after surgery has been initiated and the deficiencies of the previous measurements have been experimented in situ, the implant set that adapts to the actual need of the particular case.

With regard to the angles obtained by means of wedged pieces, another preferred embodiment to achieve said angles consists in fixing the bars and tubes in a leaned way in relation with the flat members supporting them. For example, bars might be fixed at 8° with regard to the member supporting it and tubes at -8°, including any other required value, thus achieving the desired angular correction, and simplifying the production of supporting pieces. This angular correction system is more preferred for the two-piece implant. It is not convenient to bend the bars. They should be leaned instead since its curvature would be generating lateral efforts endangering the system stability.

With regard to vertebral separation bars, further to the possibility of having pieces of different bar lengths, another preferred embodiment of the invention comprises the alternative of shortening the bars by their ends in order to adapt their lenghts. Said cuts usually originate small flanges which obstruct the free movement between the bars and tubes.

In order to overcome said problem, the hole composed of a tube and the hole in the piece supporting it and the hole in the supporting piece will form a hole with two different diameters: the diameter corresponding to the upper section (closest to the vertebral plate) will be slightly bigger than the diameter of the bottom section of the tube. In this way, the possible flanges will not obstruct the introduction of the bars into the tubes, and the process for the final adjustment of the separation between the opposite faces of the implant may be normally continued. This construction makes it possible that the set keeps the firmness for which it has been designed by keeping the diameter o the bottom section of the hole containing the bars adjusted to the diameter of the latter.

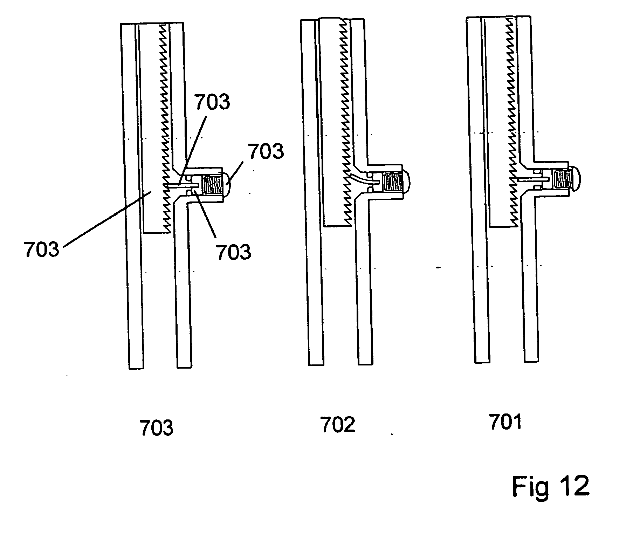

Another preferred embodiment to overcome the problem of flanges produced by the cut, consists in using diametrically grooved bars and said grooving may be either smooth or indented. The bar cut is carried out on the smallest diameter slits. In the case of an indented grooving, it has a second application since it may be used as a prefixation system of the implant set height. It is achieved by adding a semiflexible tooth or feather oriented towards the interior of the telescopic adjusting piece. Figure 12 illustrates one of the various possibilities of this preadjusting system.

The obstruction device comprises an unidirectional flexible feather, fixed in the interior of the tube, at the height of the lateral hole in said tube, in its upper part, and is flexible towards the separation direction of the pieces. Among the different techniques used so that said feather is flexible in an only one direction, the use of a flexible curved metal sheet is described. Said curvature is obtained by fixing said metal sheet to the adjusting hole 25, in its upper section. In this way, the convex side efforts flex the feather thereby enabling the separating sliding of the pieces, and the concave side efforts resist said flexion by obstructing the bar at the desired height.

Said device will enable the sliding tending to separate the pieces and will obstuct the sliding tending to reduce the distance between the pieces. In this way, the surgeon, when placing the implant, may initiate the separation of the upper and lower pieces with the corresponding instruments, and once the desired separation has been reached, the surgeon may definitely adjust it with the screws without worrying about keeping the separation with his hands or by means of accessory instruments which may obstruct the operation field and make the adjustment handlings difficult.

Likewise, a tooth system may be used, which tooth is a triangulate with a rectangle, which horizontal side is perpendicular to the tube and is the bottom side of the triangle, and which vertical side is parallel to the tube. Of course, the tube includes an indentation of a triangulate rectangular shape where the horizontal side is the upper side and the oblique side is the bottom side. The upper side and the oblique side of the tooth, when pressure is exerted on the bottom and oblique sides of the indentation teeth of the tube, enable the sliding of the indented tube due to the flexion possibility of the sheet supporting the tooth and the space of the horizontal tube where the bar fixation screws will be afterwards placed. Once the desired height has been reached, the bars remain obstructed when contacting the two horizontal surfaces of the respective teeth. For a definite fixation of the height, the fixation screw 20 is placed in the threaded hole 25, which will exert pressure on the tooth against the bar, thereby obtaining an excellent fixation for the device.

Therefore, an object of this invention is an implant comprised by two pieces with an automatic height prefixing system, by means of prefixing means between the bars and the tubes, as well as an angular correction system consisting in fixing the bars and tubes leaning towards the flat member supporting them. Furthermore, the bars may be cut at the necessary height, in the diametrical grooves having a small diameter to be used for said purpose.

In order to free the system from the exclusive manual ability of the professional, the implant may have incuts and discontinuities in is frame as illustrated in Figure 11 A for thoracic implants and in Figure 15 for cervical implants. Said configuration is not illustrated but it is extensible to the lumbar implant.

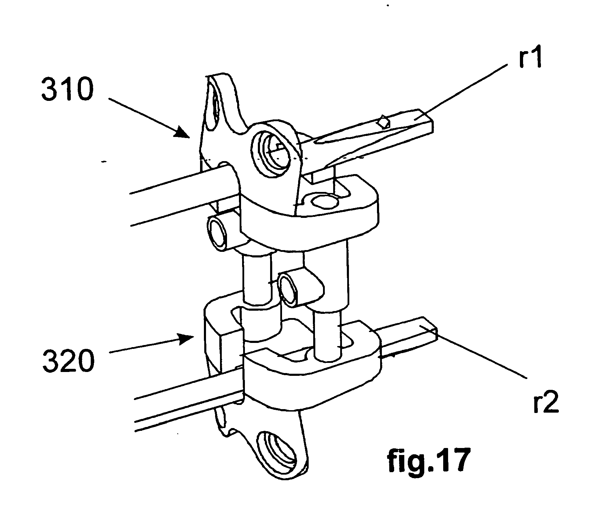

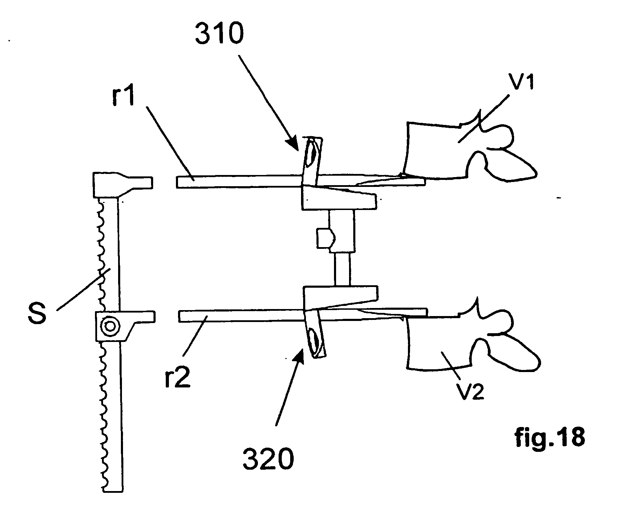

Figure 17 illustrates the use of an instrument for the implant placement. The instrument consists in nippers or "scissors" that include stops near its ends R1 and R2. The upper and lower pieces supported with their incuts and they slid along the nippers arms up to the stops. Said stops contact the lateral wall of the vertebrae, as shown in Figure 18. In this way, the implant is presented. The desired separation of both the implant and the vertebrae is obtained by exerting pressure with the nippers and then the implant is slipped until it is positioned in the intervertebral space. The nippers may be easily removed by reducing the pressure thereon and owing to the discontinuity of the horizontal frames and, in this way, the piece is ready for its final adjustment. The prefixing system enables the nippers removal without causing any collapse of the set. In this way, the surgeon will have a free operation field to make the definite adjustments by means of screws used for said purpose.

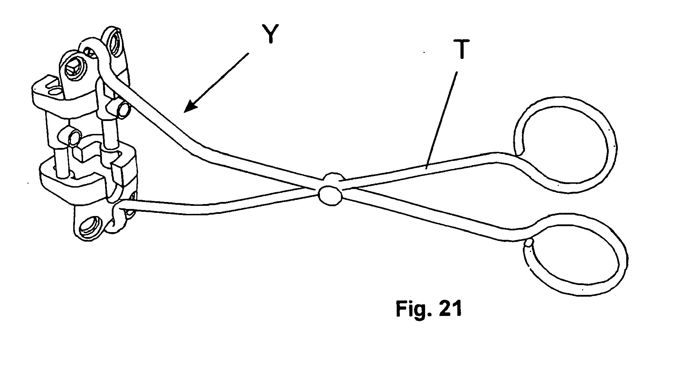

Figure 21 illustrates alternative nippers for the placement of an implant by standard methods.

An implant according to the present invention may be placed by the following surgical method:
1. By an intervention anterior to the column, the injured vertebral bodies with their respective disks are removed and the bone surfaces are prepared by removing its cartilage in its entirety and leaving the bone exposed.
2. Assembly of the four-piece implant: it is carried out outside the patient. Assembly of pieces 1 and 2: an accessory piece 1 is selected, which angle must be suitable for its adaptation to the upper vertebra V1, and piece 2 is put thereon causing the anterior parts 11 and 21 and rear parts 12 and 18 to coincide with each other. In this way, pieces 1 and 2 coincide as well as their four holes 16 with the four holes 27, on the one hand, and the four holes 17 coincide with the four holes 18, on the other. Screws 10 are placed in the holes 17, and they are screwed in the threaded holes 28, and pieces 1 and 2 are thereby joined forming a set 1-2 of the upper end of the implant.
   Assembly of pieces 3 and 4: A piece 4 is selected which must be suitable for the angle of the lower vertebra V2 where it will rest on and it is put on top causing its anterior edges 31 and 41 to coincide with each other, on the one hand, and the rear edges 32 and 42 to coincide with each other, on the other hand. Four screws 10 are placed through the holes 46 and they are also threaded in the threaded holes 37, thereby forming a set 3-4 of the bottom end of the implant.
   In this way, the two ends of implant I are obtained: on the one hand, set 1-2, and on the other hand, set 3-4, and when the ends 34 of the bars 35 are passed through the tubes 231, 232 and the tubes 23, the set 1-2 is fit by its end in set 3-4 by the other end so that the surgeon has in his side the extensions 15 of piece 1, and extension 45 of piece 4, as well as the four screws 20 of the telescopic fixation system. Two screws 20 are adjusted in the tubes 24, and the other two screws 20 are adjusted in tubes 26, which are oriented at an angle convergent on the space between the two tubes 24, in order to enable the adjustment by means of a screwdriver placed between both tubes 24.
3. Placement of the implant between two lumbar vertebrae: with implant I assembled in said way, and with the ends 34 of the bars 35 surpassing set 1-1, bars 35 are cut by their ends 34, two centimeters shorter than the spinal column defect, measuring the distance between the bottom edge of piece 4 and the ends 34 of bars 35. The implant is placed in the defect of the spinal column, thereby leaving set 3-4 resting on the lower vertebra V2 and the perpendicular bars 35 and between vertebrae V1 and V2 with their ends 34 under the upper vertebra V1, so that the anterior part of vertebrae V1 and V2 coincide with the anterior edges 11, 21, 31 and 41, and the rear part of the vertebral bodies V1 and V2 coincide with edges 12, 28, 32 and 42. In this way, the set 3-4 rests on vertebra V2 by the inside of its perimeter and with extension 45 resting on its outer face, and set 1-2 is in a middle point of bars 35, which ends face vertebra V1, thus being its projection within its perimeter. If necessary, in order to centralize sets 1-2 and 3-4 in the center of vertebrae V1 and V2, it may be necessary to make a small resection on the edges of both vertebrae, aiming to adapt the shape of the lateral extensions 14 and 44, as shown in Figure 7B. Afterwards, separation of the ends formed by sets 1-2 and 3-4 is made manually or by means of separating instruments, and when freely slipping along the bars 35 the set 1-2, the separation of the platforms of the ends of implant I occurs, which turns from the position seen in Figure 10A to the final position seen in Figure 10B, the set 1-2 being applied by the inside of the upper vertebra V2 perimeter and its lateral extension 15 applied to the outer face. Screws 20 are placed and fixed in tubes 231, 232, and the two tubes 23, which fix the telescopic system, by using a screwdriver, oriented towards the screws 20 direction in order to block the telescopic system as shown in Figure 9. The implant was thus placed straining its ends against the vertebrae. Screws 30 are placed through holes 18, and screws 301 are placed through holes 19 for piece 1, and vertebrae are fixed, for piece 4, screws 30 are placed through holes 47 and 301 for holes 48 and they are fixed to the vertebrae. In this way, the implant I mount to the vertebral defect between two lumbar vertebrae V 1 and V2 is finished.
   A variant in the coupling is used to be placed between a lumbar vertebra V1 from the top and the sacral bone S from the bottom. Owing to the different anatomical structures of the lumbar vertebra I and the sacral bone S, the sacral bone S requires a supporting piece 5, which is adjusted to piece 3 with screws 10 which penetrate into the holes 55 and are threaded and adjusted in holes 37. The mount is then carried out in another preferred embodiment with pieces 1, 2, 3 and 5 coupling, which for its accommodation in the spinal column defect between vertebrae V1 and S, has a mechanism identical to the above-described mechanism for the accommodation of implant I.
4. Once the implant has been placed, the whole space between the vertebrae is filled in with bone grafts and bioactive materials to form the bone callus which will include the implant thereby obtaining the definite mount fixation.

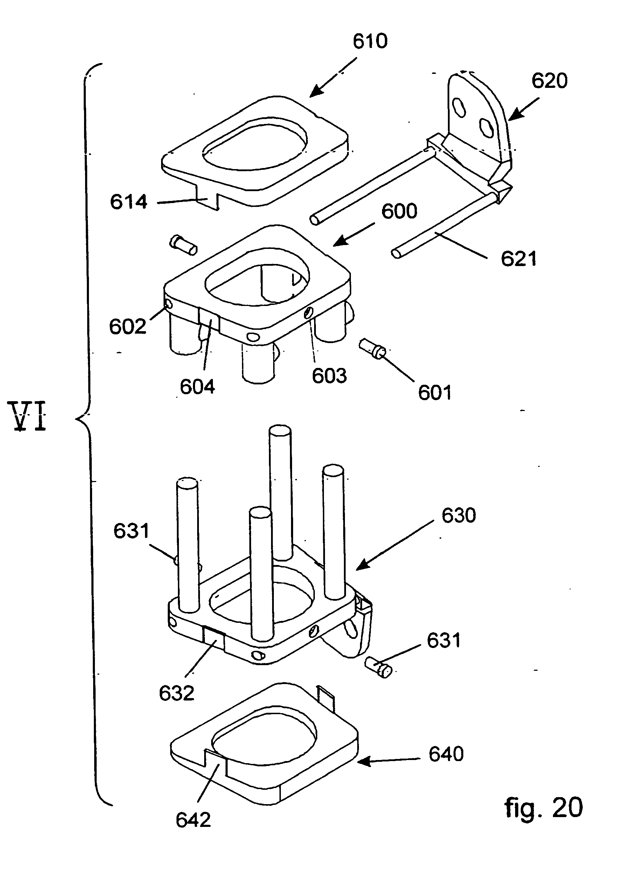

Another preferred embodiment of this invention, and with relation to the way the implant pieces couple, is illustrated in Figure 20. For the 4-piece implant, the upper vertebral supporting piece is divided into two subpieces: one angular correction subpiece 610, which is adjusted to the telescopic adjusting piece 600 by means of flat projections 614 which fit in fitting spaces 604. In the same way, the bottom vertebral supporting piece is divided into two subpieces of identical configuration. The second subpiece 620 consists of the vertical extension including two horizontal bars 621, which will be introduced in the holes 602 of the new telescopic adjusting piece 600. The subpiece 620 is fixed in the piece 600 by means of the bar adjustment with screws 601, which are introduced in the lateral holes 603 of the telescopic adjusting piece 600. A configuration of this nature enables the reduction of the number of manual adjustments to be made to make the implant, and as a consequence only the adjustment of 4 screws is required for the assembly of all the pieces.

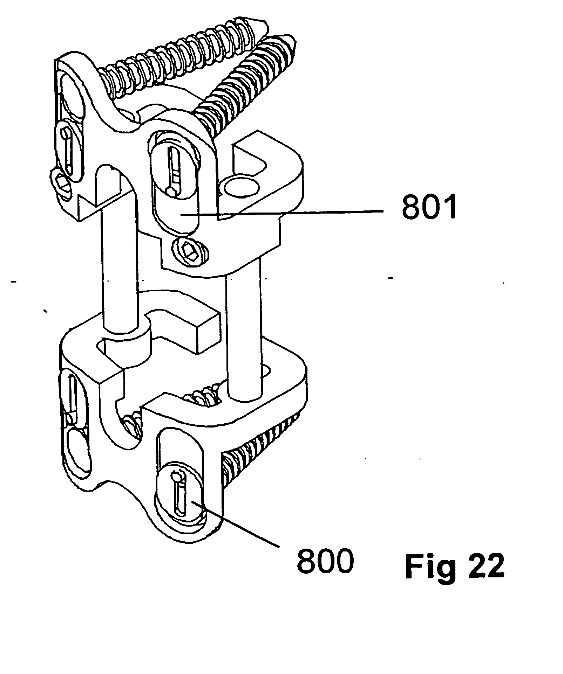

Another variant may be presented in the blocking system of the screws that are introduced in the body. This variant consists of slipping stops in order to prevent the fixed screw from a longitudinal displacement, as shown in Figure 22. A grrove is made on the lateral extension where said screws are, said groove having a rectangular shape with upper and bottom semicircular ends. One end of the groove will have the hole for the screw passage. The other end will have a circular shaped slipping stop and a groove to serve as a guide for the headed-bar type projection. Said guide-groove is vertically oriented in order to allow the screw head to slip and continues with an extension towards the circumference direction which allows it to rotate and thereby preventing the stop from a vertical slipping.

Another variant related to the orientation of the tube holes receiving the fixation screw 20 is illustrated in Figures 9A and 9 B, wherein the longitudinal axis directions of the tube holes located at the longest distance from the surgeon's access converge on a point located in the middle of the side defined by the two tubes most accessible to the surgeon, so that said orientation enables the introduction of instruments for adjusting screws in the less accessible tubes.

Figure 22 further illustrates a variant wherein the telescopic adjusting tube is not necessary by means of a hole in the horizontal member of the upper piece of the implant. Said variation is obtained by increasing the width of the horizontal member in the section of the original location of the tubes. The fixing screws are placed in holes made in the lateral faces of the horizontal member which height has been modified.

After having described and invented the several preferred embodiments of this invention, which are described in the priority document, there followed the step of prototype making and tests in relation with the mechanical loads and practicity in the use of the device. A series of adaptations and revisions, which are described hereinbelow, has arisen from said tests.
After having verified the mechanical resistance of the structure and the materials used at present for this kind of device, it has been determined that the model for the lumbar area, the dorsal area and the cervical area will comprise two bars with their corresponding tubes. With regard to the parts forming the supports of the tubes, and similar to the proposal as an alternative embodiment for the cervical area device, it has been decided to use a top part instead of a supporting part and an adjusting part. Figure A shows a two-bar device to be applied to the lumbar area. Figure D shows the same device to be applied to the cervical area. In the same way, the thoracic area proceeds respecting the substantially triangular shape of said area vertebrae.
The top part (1010. Fig. A) is a vertebral supporting frame. From their short sides facing each other, tubes that will receive the bars emerge. Said tubes enable the bar slipping for the adjustment of device height. The bars will then be fixed to the tubes by means of fastening means, preferably screws entering through screwed holes located at the side walls of the tubes and in different spatial plans, as indicated in Fig. E (1060), which perpendicular direction must be free to enable the screws adjustment. The side opposite the vertebrae has a wedge-like shape and the oblique side of the wedge faces the vertebral plate. The tubes of the top part are not located exactly in the side centers but are moved forward in order to make use, if necessary, of the remaining bone of the injured vertebra.

A removable jig (1040 Fig. B) is provided having the shape of the corresponding frame and a wedge-like shape which angles are predetermined. The jig has protuberances on its lower surface which fix in the corresponding incuts (1042) located on the inner perimeter of the ring of the vertebral supporting frame. It further has an incut (1043) on the outer perimeter of the part in order to enable the removal of the jig.

The jig comprises on its top surface a series of orientated wings offering resistance against slipping. The material recommended consists in titanium microgranules.

Furthermore, the top part and the bottom part may have inclinations as indicated in Figure C (1011, 1012, 1013). The inclinations may be formed by any angle and parts of 0 degree, 5 degrees and 9.5 degrees are proposed. Said inclinations in combination with the jigs, which wedge-like shape may form angles of 0 degree, 1 degree, 2 degrees and 3 degrees, provide the set with a wide variety of inclinations suitable for the angular correction between the vertebrae.

In Figure E, the part (1050) shows an orientated-wing type surface directly applied onto its surface instead of using the jig.

The vertebral supporting frame also has a vertical extension on the end of its short sides. Said vertical extension has a curved shape that is adapted to the lateral vertebral face. It also includes two screwed holes and orientated in different spatial plans in order to achieve a spatial fixing

With reference to the vertical extension, there exists a horizontal slipping with regard to the frame original edge for its correct location and fixing as indicated in Fig. C (1090 and 1018). Said slippings are at predetermined distances.

With regard to the lower part (1020 Fig. A), which is the lower vertebral supporting frame, the same considerations as the ones for the top part may be applied, except that it lacks the tubes through which the bars slip (1035) and instead it has small threaded tubes wherein the bars are screwed. The frame shape, the fastening means, the slipping of the threaded tubes and of the vertical member are all in accordance with the top part It is also possible to use the corresponding angular correction jig, which may have an inclination different from the inclination of the top part according to the desired correction.

With regard to the bars (1035) that coil around the bottom part and get inserted into the tubes of the top part, they are substantially cylindrical and have a threaded bottom end and top end having a nut shape in order to facilitate its coiling around part B. The bars further have a mark which determines the extent they may slip within the receiving tube of part A. The bars have predetermined heights (Fig. C 1036/37/38/39) and have been designed to provide a height adjustment of 11 mm.

Another possibility for the present invention consists in placing a screen in the back area. Therefore, the top part has a hole (1090) as indicated in Figure E, which may hold said screen.

With regard to the facing letter "E" (1070 Figure E), characteristic of the adaptation to the cervical area of the present invention, the possibility of having the ends joined has been considered in order to provide the whole set with stability as indicated in Fig. E (1080). This modification does not enable the use of the proposed placing tools but enables the use of standard tools.

## Claims

1. An implant for vertebral replacement comprised by inner supports to the vertebral plates (1, 4, 110, 140, 410, 440, 310, 320, 315), vertebral separating means and vertical members for adjusting the implant to the corresponding vertebral faces (2, 3, 120, 130, 420, 430), wherein the piece of the inner support implant emulates the shape of the cortical tissue area of the vertebral plate thereby forming a frame and leaving the spongy tissue area of the vertebral plate free;
**characterized in that**
the vertebral separating means comprises a set of bars (35, 1039, 1038, 1037, 1036) and a set of tubes (23, 231, 232), corresponding with each other, the outer diameters of the bars and the inner diameters of the tubes having a size enabling the adjusted slipping of the bars into the tubes and the fixation thereof at a height determined by fixing means.

2. The implant of claim 1, **characterized in that** the superior piece and the inferior piece allow the insertion of a removable jig (1040), having a shape corresponding to the frame and a wedge-like shape which angles are predetermined; the pieces include incuts for purposes of insertion and removal of the jig.

3. The implant of claim 1, **characterized in that** the horizontal part of the superior and inferior pieces may be inclined with respect to the vertical members, to provide angular correction to the spine.

4. The implant of claim 1, **characterized in that** both the tubes (23, 231, 232) and the bars (35, 1039, 1038, 1037, 1036) are fixed in middle points in at least two of the sides forming the inner support.

5. The implant of claim 1, **characterized in that** both the tubes (23, 231, 232) and the bars (35, 1039, 1038, 1037, 1036) are fixed between the center and the posterior area points in just two opposite sides of the inner support.

6. The implant of claim 1, **characterized in that** the bar diameter is lower than 8 mm.

7. The implant of claim 4, **characterized in that** the bar diameter is of 4 mm.

8. The implant of claim 1, **characterized in that** the bars are grooved in order to obtain diameter sections lower than the bar predetermined diameter to cut the bars.

9. The implant of claim 8, **characterized in that** the bar grooving is indented and the corresponding tube includes a device (703) for obstructing the bar slipping into the tube towards a direction preferably corresponding to the approach of the vertebral separating pieces and the telescopic adjustment.

10. The implant of claim 1, **characterized in that** it is composed of four pieces:
an upper supporting piece (1) having a horizontal member (11) and a vertical extension (14) for its adjustment to the lateral vertebral face by means of screws;
a telescopic adjusting piece (2) having a flat member (21) suitable for its adjustment to the upper supporting piece and at least two vertical extensions being tubes (23, 231, 232) perpendicular to the horizontal member;
a piece of vertebral separation (3) comprising a flat member (31) and a number equal to the telescopic adjusting piece of vertical extensions being bars (35) perpendicular to the horizontal member which will be introduced into the tubes of the telescopic adjusting tubes; and
a bottom supporting piece (4) having a horizontal member (41) and a vertical extension (45) for its adjustment to the vertebral lateral face by means of screws.

11. The implant of claim 10, **characterized in that** the horizontal members (11, 41) of the upper and bottom supporting pieces (1, 4), and the horizontal members of the telescopic adjusting piece, and the vertebral separating piece have the shape selected from the group defined by the vertebral plate cortical areas of the lumbar vertebrate with a vertebral ring shape, ring-shaped cervical vertebrae formed by two letters "E" facing each other and thoracic vertebrae with a triangulate ring shape.

12. The implant of claim 10, **characterized in that** the bottom supporting piece (4) is composed of a flat member and a flat extension which is adapted to the sacral bone, said flat member having an adaptable rectangular ring shape and adjustable to the vertebral separating piece.

13. The implant of claim 10, **characterized in that** the upper and bottom supporting pieces (1, 4) are a horizontal wedged member forming an angle for the restoration of the spinal curvature.

14. The implant of claim 10, **characterized in that** the union of pieces to each other is manually made with adjusting and fixing screws.

15. The implant of claim 1, **characterized in that** it is comprised by two pieces: an upper supporting and telescopic adjusting piece (1010, 310) having a horizontal member and a vertical extension for its adjustment to the vertebral lateral face by means of screws, and at least two vertical extensions being tubes perpendicular to the horizontal member; a bottom supporting and vertebral separating piece (1020, 320) having a flat member and a number equal to the telescopic adjusting piece of vertical extensions being bars perpendicular to the horizontal member, which will be introduced into the tubes of the telescopic adjusting piece, and a vertical extension for its adjustment to the vertebral lateral face by means of screws.

16. The implant of claim 15, **characterized in that** the horizontal members of said pieces have the shape selected from the group defined by the cortical areas of the vertebral plates of lumbar vertebrae having a vertebral ring shape, cervical vertebrae with a shape formed by two letters "E" facing each other, and triangulate ring shaped thoracic vertebrae.

17. The implant of claim 15, **characterized in that** the horizontal members of the pieces are comprised by a flat member and a flat extension which is adapted to the sacral bone, with a rectangular ring shape.

18. The implant of claim 15, **characterized in that** the piece horizontal members are wedged shaped forming an angle for the restoration of the spinal curvature.

19. The implant of claim 10, **characterized in that** the union between supporting pieces and vertebral separation and telescopic adjusting pieces have reciprocal fitting mechanisms.

20. The implant of claims 10 and 15, **characterized in that** the vertebral supporting surfaces of said pieces have surfaces which are not smooth.

21. The implant of claim 20, **characterized in that** the vertebral supporting pieces have wrinkled surfaces.

22. The implant of claim 20, **characterized in that** the vertebral supporting pieces have indented surfaces.

23. The implant of claims 10 and 15, **characterized in that** the lateral extensions adjusting against the lateral vertebral face enable the insertion of screw oblique to the longitudinal axis of the vertebrae.

24. The implant of claims 10 and 15, **characterized in that** the vertebral supporting piece and the vertebral separating piece are comprised by a unique piece having three flat members forming at least obtuse angles between them, and its bottom member includes vertical bars; its middle flat member includes holes for the passage of screws, thus turning this configuration suitable for its placement in the axis bone.

25. The implant of claims 10 and 15, **characterized in that** the vertical extensions of the vertebral supporting pieces include a stop system (701, 702, 703) to prevent the screws that penetrate into the vertebral body from their longitudinal slipping.

26. The implant of claim 25, **characterized in that** said stop system (701, 702, 703) is comprised by a groove in the lateral extension where there are holes for the screws to penetrate into the vertebral body, said groove having a rectangular shape with upper and lower semicircular ends; an end of the groove will have a hole for the passage of the screw; the other end will have a slipping stop with a circular shape and a groove which serves as a guide to the headed bar type projection; and said guide-groove is vertically orientated in order to enable the slipping towards the screw head and continues with an extension following the direction of the circumference which enables it to rotate, thus avoiding the vertical slipping of the stop.

27. The implant of claim 1, **characterized in that** it is composed of 6 pieces:
an upper supporting piece and a bottom supporting piece having a horizontal member and which are indistinctly fixed to a telescopic adjusting and vertebral separation piece, by means of vertical projecting sheets that fit in lateral grooves of the telescopic adjusting or vertebral separation piece; an upper vertical extension piece and a bottom adjusting piece for their adjustment to the vertebral lateral face by means of screws, which includes holes arranged for said purpose and which are adjusted to the telescopic adjusting or vertebral separation piece by means of two horizontal bars, which insert in the corresponding horizontal holes in the telescopic adjusting or vertebral separating piece, which final fixation is carried out by means of screws fit in holes located in the lateral faces of the telescopic adjusting and vertebral separating pieces;
a telescopic adjusting piece composed of a flat member suitable for the adjustment thereof to the upper supporting piece, which includes grooves in at least tow of its lateral faces to be fit in the vertebral supporting pieces, and at least two holes horizontally extend in order to contain two tubes of the vertical extension piece, and at least two vertical extensions being tubes perpendicular to the horizontal member;
a vertebral separating piece composed of a flat member and a number equal to the telescopic adjusting piece, of vertical extensions being bars perpendicular to the horizontal member and said bars will be introduced into the tubes of the telescopic adjustment piece; said vertebral separating piece includes grooves in at least two of its lateral faces to be fit in vertebral supporting pieces, and at least two holes horizontally extending to contain two tubes of the vertical extension piece.

28. The implant of claims 1, 10, 15 and 27, **characterized in that** the bars and tubes are fixed with a leaning in relation to the pieces holding them, thereby providing the implant set with a curvature.

29. The implant of claims 1, 10, 15 and 27, **characterized in that** it includes incuts and discontinuities for the use of nippers with stops that enable the vertebral separation and the placement of the implant by means of said instrument.

30. The implant of claim 2, **characterized in that** the removable jig includes on its top surface a series of orientated wings offering resistance against slipping.

31. The implant of claim 2, **characterized in that** the wedge-like shape of the jig may form angles of 0 degrees, 1 degree, 2 degrees and 3 degrees.

32. The implant of claim 1, **characterized in that** both the tubes and the bars are fixed in the vertices of the inner supports.

33. The implant of claim 3, **characterized in that** the inclination of horizontal part of the superior and inferior pieces with respect to the vertical members, to provide angular correction to the spine is selected from the following values: 0 degrees, 5 degrees and 9,5 degrees.

34. The implant of claim 1, **characterized in that** the vertical extension has a curved shaped to adjust the device to the lateral vertebral face.

35. The implant of claim 1, **characterized in that** the vertical extension has an horizontal slipping at predetermined distances.

36. The implant of claim 1, **characterized in that** the superior piece includes a removable optional screen fixed by a screw to the piece to be located in the anterior area to avoid the migration of bone out of the vertebral area.

37. The implant of claims 1, 10, 15 and 27, **characterized in that** includes just two bars and two corresponding tubes for separating means.

38. The implant of claim 2, **characterized in that** the jig is made of titanium microgranules.

39. The implant of claims 1, 10, 15 and 27 **characterized in that** the bars and tubes allows an adjustment in height of 11 mm.

## Patentansprüche

1. Implantat für Wirbelkörperersatz, bestehend aus inneren Trägern für die Wirbelkörperplatten (1, 4, 110, 140, 410, 440, 310, 320, 315), Wirbelkörpertrenneinrichtungen und vertikalen Elementen zum Einstellen des Implantats auf die entsprechenden Wirbelkörperflächen (2, 3, 120, 130, 420, 430), wobei das Stück des inneren Trägerimplantats die Form des Rindengewebebereichs der Wirbelkörperplatte emuliert, wodurch ein Rahmen gebildet wird und der schwammige Gewebebereich der Wirbelkörperplatte frei gelassen wird;
**dadurch gekennzeichnet, dass**
die Wirbelkörpertrenneinrichtung einen Satz Stäbe (35, 1039, 1038, 1037, 1036) und einen Satz Röhren (23, 231, 232), die miteinander korrespondieren, umfasst, wobei die Außendurchmesser der Stäbe und die Innendurchmesser der Röhren eine Größe aufweisen, die das eingestellte Gleiten der Stäbe in die Röhren hinein und die Befestigung davon in einer durch Befestigungseinrichtungen bestimmten Höhe ermöglicht.

2. Implantat nach Anspruch 1, **dadurch gekennzeichnet, dass** das obere Stück und das untere Stück das Einfügen einer abnehmbaren Einspannvorrichtung (1040) mit einer Form entsprechend dem Rahmen und einer keilförmigen Form, deren Winkel vorgegeben sind, ermöglichen; wobei die Stücke Einschnitte für Zwecke des Einfügens und Abnehmens der Einspannvorrichtung umfassen.

3. Implantat nach Anspruch 1, **dadurch gekennzeichnet, dass** der horizontale Teil des oberen und unteren Stücks in Bezug auf die vertikalen Elemente geneigt sein kann, um Winkelkorrektur für die Wirbelsäule bereitzustellen.

4. Implantat nach Anspruch 1, **dadurch gekennzeichnet, dass** sowohl die Röhren (23, 231, 232) als auch die Stäbe (35, 1039, 1038, 1037, 1036) in Mittelpunkten in wenigstens zwei der Seiten, die den inneren Träger bilden, befestigt sind.

5. Implantat nach Anspruch 1, **dadurch gekennzeichnet, dass** sowohl die Röhren (23, 231, 232) als auch die Stäbe (35, 1039, 1038, 1037, 1036) zwischen der Mitte und den Hinterflächenpunkten in lediglich zwei einander gegenüberliegenden Seiten des inneren Trägers befestigt sind.

6. Implantat nach Anspruch 1, **dadurch gekennzeichnet, dass** der Stabdurchmesser geringer als 8 mm ist.

7. Implantat nach Anspruch 4, **dadurch gekennzeichnet, dass** der Stabdurchmesser 4 mm beträgt.

8. Implantat nach Anspruch 1, **dadurch gekennzeichnet, dass** die Stäbe genutet sind, um Durchmesserabschnitte zu erzielen, die niedriger sind als der vorgegebene Stabdurchmesser, um die Stäbe zu schneiden.

9. Implantat nach Anspruch 8, **dadurch gekennzeichnet, dass** die Stabnutung eingekerbt ist und die entsprechende Röhre eine Vorrichtung (703) umfasst, um das Hineingleiten des Stabs in die Röhre in eine Richtung, die vorzugsweise der Annäherung der Wirbelkörpertrennstücke und der Teleskopeinstellung entspricht, zu blockieren.

10. Implantat nach Anspruch 1, **dadurch gekennzeichnet, dass** es aus vier Stücken besteht.
- ein oberes Tragstück (1) mit einem horizontalen Element (11) und einer vertikalen Verlängerung (14) für seine Einstellung auf die seitliche Wirbelkörperfläche mit Hilfe von Schrauben;
- ein Teleskopeinstellstück (2) mit einem flachen Element (21), das für seine Einstellung auf das obere Tragstück geeignet ist, und wenigstens zwei vertikalen Verlängerungen, die Röhren (23, 231, 232) senkrecht zu dem horizontalen Element sind;
- ein Wirbelkörpertrennstück (3), umfassend ein flaches Element (31) und eine dem Teleskopeinstellstück entsprechende Anzahl vertikaler Verlängerungen, die Stäbe (35) senkrecht zu dem horizontalen Element sind, die in die Röhren der Teleskopeinstellröhren eingeführt werden; und
- ein unteres Tragstück (4) mit einem horizontalen Element (41) und einer vertikalen Verlängerung (45) für seine Einstellung auf die Wirbelkörperseitenfläche mit Hilfe von Schrauben.

11. Implantat nach Anspruch 10, **dadurch gekennzeichnet, dass** die horizontalen Elemente (11, 41) des oberen und unteren Tragstücks (1, 4) und die horizontalen Elemente des Teleskopeinstellstücks und das Wirbelkörpertrennstück die Form aufweisen, die aus der Gruppe gewählt ist, die durch die Wirbelkörperplattenrindenbereiche des Lendenwirbels mit einer Wirbelkörperringform, ringförmige Halswirbel, die durch zwei einander gegenüberliegenden Buchstaben "E" gebildet werden, und Brustwirbel mit einer Dreiecksringform definiert wird.

12. Implantat nach Anspruch 10, **dadurch gekennzeichnet, dass** das untere Tragstück (4) aus einem flachen Element und einer flachen Erweiterung, die auf das Kreuzbein eingestellt ist, besteht, wobei das flache Element eine anpassbare Rechtecksringform aufweist und auf das Wirbelkörpertrennstück einstellbar ist.

13. Implantat nach Anspruch 10, **dadurch gekennzeichnet, dass** das obere und das untere Tragstück (1, 4) ein horizontales Keilelement sind, das einen Winkel für die Wiederherstellung der Wirbelsäulenkrümmung bildet.

14. Implantat nach Anspruch 10, **dadurch gekennzeichnet, dass** die Verbindung von Stücken miteinander manuell mit Einstell- und Befestigungsschrauben erfolgt.

15. Implantat nach Anspruch 1, **dadurch gekennzeichnet, dass** das es aus zwei Stücken besteht: einem oberen Trag- und Teleskopeinstellstück (1010, 310) mit einem horizontalen Element und einer vertikalen Erweiterung für seine Einstellung auf die Wirbelkörperseitenfläche mit Hilfe von Schrauben und wenigstens zwei vertikalen Verlängerungen, die Röhren senkrecht zu dem horizontalen Element sind; einem unteren Trag- und Wirbelkörpertrennstück (1020, 320) mit einem flachen Element und einer dem Teleskopeinstellstück entsprechenden Anzahl vertikaler Verlängerungen, die Stäbe senkrecht zu dem horizontalen Element sind, die in die Röhren des Teleskopeinstellstücks eingeführt werden, und einer vertikalen Verlängerung für seine Einstellung auf die Wirbelkörperseitenfläche mit Hilfe von Schrauben.

16. Implantat nach Anspruch 15, **dadurch gekennzeichnet, dass** die horizontalen Elemente der Stücke die Form aufweisen, die aus der Gruppe gewählt ist, die durch die Rindenbereiche der Wirbelkörperplatten der Lendenwirbel mit einer Wirbelkörperringform, ringförmige Halswirbel mit einer Form, die durch zwei einander gegenüberliegende Buchstaben "E" gebildet wird, und dreiecksringförmige Brustwirbel definiert wird.

17. Implantat nach Anspruch 15, **dadurch gekennzeichnet, dass** die horizontalen Elemente der Stücke aus einem flachen Element und einer an das Kreuzbein angepassten flachen Erweiterung mit einer Rechtecksringform bestehen.

18. Implantat nach Anspruch 15, **dadurch gekennzeichnet, dass** die horizontalen Stückelemente keilförmig sind und einen Winkel für die Wiederherstellung der Wirbelsäulenkrümmung bilden.

19. Implantat nach Anspruch 10, **dadurch gekennzeichnet, dass** die Verbindung zwischen Tragstücken und Wirbelkörpertrenn- und Teleskopeinstellstücken wechselseitige Einpassmechanismen aufweisen.

20. Implantat nach den Ansprüchen 10 und 15, **dadurch gekennzeichnet, dass** die Wirbelkörpertragflächen der Stücke Flächen aufweisen, die nicht glatt sind.

21. Implantat nach Anspruch 20, **dadurch gekennzeichnet, dass** die Wirbelkörpertragstücke faltige Flächen aufweisen.

22. Implantat nach Anspruch 1, **dadurch gekennzeichnet, dass** die Wirbelkörpertragstücke eingekerbte Flächen aufweisen.

23. Implantat nach den Ansprüchen 10 und 15, **dadurch gekennzeichnet, dass** die seitlichen Verlängerungen, die sich gegen die seitliche Wirbelkörperfläche einstellen, das Schraubeneinfügen schräg zu der Längsachse der Wirbelkörper ermöglichen.

24. Implantat nach den Ansprüchen 10 und 15, **dadurch gekennzeichnet, dass** das Wirbelkörpertragstück und das Wirbelkörpertrennstück aus einem einzigen Stück bestehen, das drei flache Elemente, die wenigstens stumpfe Winkel zwischen ihnen bilden, aufweist und sein unteres Element vertikale Stäbe umfasst; wobei sein mittleres flaches Element Löcher für den Durchgang von Schrauben umfasst, wodurch diese Gestaltung die Eignung für ihre Platzierung in dem zweiten Halswirbelkörper erlangt.

25. Implantat nach den Ansprüchen 10 und 15, **dadurch gekennzeichnet, dass** die vertikalen Verlängerungen der Wirbelkörpertragstücke ein Anschlagsystem (701, 702, 703) umfassen, um das Längsgleiten der Schrauben, die in den Wirbelkörperkörper eindringen, zu verhindern.

26. Implantat nach Anspruch 25, **dadurch gekennzeichnet, dass** das Anschlagsystem (701, 702, 703) aus einer Nut in der seitlichen Verlängerung besteht, in der sich Löcher für die Schrauben zum Eindringen in den Wirbelkörperkörper befinden, wobei die Nut eine rechtwinklige Form mit oberem und unterem halbkreisförmigen Ende aufweist; wobei ein Ende der Nut ein Loch für den Durchgang der Schraube aufweist; das andere Ende einen Gleitanschlag mit einer Kreisform und eine Nut, die als eine Führung für den Vorsprung der Art eines Stabes mit Kopf dient, aufweist; und die Führungsnut vertikal ausgerichtet ist, um das Gleiten in Richtung des Schraubenkopfes zu ermöglichen, und sich mit einer Verlängerung, die der Richtung des Umfangs folgt, fortsetzt, was ihr Drehen ermöglicht, wodurch das vertikale Gleiten des Anschlags verhindert wird.

27. Implantat nach Anspruch 1, **dadurch gekennzeichnet, dass** es aus sechs Stücken besteht:
- ein oberes Tragstück und ein unteres Tragstück, die ein horizontales Element aufweisen und die mit Hilfe von vertikal vorstehenden Folien, die in seitliche Nuten des Teleskopeinstell- oder Wirbelkörpertrennstücks passen, nicht genau bestimmbar an einem Teleskopeinstell- und Wirbelkörpertrennstück befestigt sind; ein oberes vertikales Verlängerungsstück und ein unteres Einstellstück für ihre Einstellung auf die Wirbelkörperseitenfläche mit Hilfe von Schrauben, wobei dies Löcher umfasst, die für den Zweck angeordnet sind und die mit Hilfe von zwei horizontalen Stäben, die sich in die entsprechenden horizontalen Löcher in dem Teleskopeinstell- oder Wirbelkörpertrennstück einfügen, auf das Teleskopeinstell- oder Wirbelkörpertrennstück eingestellt werden, wobei die endgültige Befestigung mit Hilfe von Schrauben durchgeführt wird, die in Löcher eingepasst werden, die sich in den seitlichen Flächen der Teleskopeinstell- und Wirbelkörpertrennstücke befinden;
- ein Teleskopeinstellstück, bestehend aus einem flachen Element, das für die Einstellung davon auf das obere Tragstück geeignet ist, umfassend Nuten in wenigstens zwei seiner seitlichen Flächen, die in die Wirbelkörpertragstücke einzupassen sind, und wenigstens zwei Löcher, die sich horizontal erstrecken, um zwei Röhren des vertikalen Verlängerungsstücks zu enthalten, und wenigstens zwei vertikale Verlängerungen, die Röhren senkrecht zu dem horizontalen Element sind;
- ein Wirbelkörpertrennstück, bestehend aus einem flachen Element und einer dem Teleskopeinstellstück entsprechenden Anzahl vertikaler Verlängerungen, die Stäbe senkrecht zu dem horizontalen Element sind, wobei die Stäbe in die Röhren des Teleskopeinstellstücks eingeführt werden; wobei das Wirbelkörpertrennstück Nuten in wenigstens zwei seiner seitlichen Flächen, die in die Wirbelkörpertragstücke einzupassen sind, und wenigstens zwei Löcher, die sich horizontal erstrecken, um zwei Röhren des vertikalen Verlängerungsstücks zu enthalten, umfasst.

28. Implantat nach den Ansprüchen 1, 10, 15 und 27, **dadurch gekennzeichnet, dass** die Stäbe und Röhren mit einer Neigung in Bezug auf die Stücke, die sie halten, befestigt werden, wodurch der Implantatsatz mit einer Krümmung versehen wird.

29. Implantat nach den Ansprüchen 1, 10, 15 und 27, **dadurch gekennzeichnet, dass** es Einschnitte und Unstetigkeiten für die Verwendung von Zangen mit Anschlägen umfasst, die die Wirbelkörpertrennung und die Platzierung des Implantats mit Hilfe des Instruments ermöglichen.

30. Implantat nach Anspruch 2, **dadurch gekennzeichnet, dass** die abnehmbare Einspannvorrichtung auf ihrer oberen Fläche eine Reihe von ausgerichteten Flügeln umfasst, die Widerstand gegen Gleiten bieten.

31. Implantat nach Anspruch 2, **dadurch gekennzeichnet, dass** die keilförmige Form der Einspannvorrichtung Winkel von 0 Grad, 1 Grad, 2 Grad und 3 Grad bilden kann.

32. Implantat nach Anspruch 1, **dadurch gekennzeichnet, dass** sowohl die Röhren als auch die Stäbe in den Scheitelpunkten der inneren Träger befestigt sind.

33. Implantat nach Anspruch 3, **dadurch gekennzeichnet, dass** die Neigung des horizontalen Teils des oberen und unteren Stücks in Bezug auf die vertikalen Elemente zur Bereitstellung von Winkelkorrektur für die Wirbelsäule aus den folgenden Werten gewählt ist: 0 Grad, 5 Grad und 9,5 Grad.

34. Implantat nach Anspruch 1, **dadurch gekennzeichnet, dass** die vertikale Verlängerung eine gekrümmte Form aufweist, um die Vorrichtung auf die seitliche Wirbelkörperfläche einzustellen.

35. Implantat nach Anspruch 1, **dadurch gekennzeichnet, dass** die vertikale Verlängerung eine horizontale Abgleitung in vorgegebenen Abständen aufweist.

36. Implantat nach Anspruch 1, **dadurch gekennzeichnet, dass** das obere Stück eine abnehmbare optionale Abdeckung umfasst, die durch eine Schraube an dem Stück befestigt ist und in dem hinteren Bereich anzuordnen ist, um das Herauswandern von Knochen aus dem Wirbelkörperbereich zu verhindern.

37. Implantat nach den Ansprüchen 1, 10, 15 und 27, **dadurch gekennzeichnet, dass** es lediglich zwei Stäbe und zwei entsprechende Röhren für Trenneinrichtungen umfasst.

38. Implantat nach Anspruch 2, **dadurch gekennzeichnet, dass** die Einspannvorrichtung aus Titanmikrogranulat hergestellt ist.

39. Implantat nach den Ansprüchen 1, 10, 15 und 27, **dadurch gekennzeichnet, dass** die Stäbe und Röhren eine Einstellung in der Höhe von 11 mm ermöglicht.

## Revendications

1. Un implant de remplacement vertébral comportant des supports internes aux plateaux vertébraux (1, 4, 110, 140, 440, 310, 320, 315), des moyens de séparation vertébrale et des membres verticaux pour l'ajustage de l'implant aux faces vertébrales (2, 3, 120, 130, 420, 430) où la pièce de l'implant de support interne imite la forme de la surface du tissu cortical du plateau vertébral constituant un cadre et en laissant libre la surface du tissu spongieux du plateau vertébral, **caractérisé en ce que** les moyens de séparation vertébrale sont constitués d'un ensemble de barres (35, 1039, 1038, 1037, 1036) et un d'ensemble de tubes (23, 231, 232), correspondants entre eux, les diamètres externes des barres el les diamètres internes des tubes ayant de dimensions telles de permettre le coulissage ajusté des barres dans les tubes et leur attachement à une hauteur déterminée moyennant des moyens d'attachement.

2. L'implant selon la revendication 1, **caractérisé en ce que** la pièce supérieure et la pièce inférieure permettent l'insertion d'un patron amovible (1040) dont la forme correspond aux surfaces des pièces et configure une cale à angles prédéterminés; les pièces présentent des encoches pour l'insertion et l'enlèvement du patron.

3. L'implant selon la revendication 1, **caractérisé en ce que** les membres horizontaux des pièces d'appui supérieur et inférieur peuvent se trouver inclinés par rapport aux membres verticaux, afin de pourvoir une correction angulaire à la colonne vertébrale.

4. L'implant selon la revendication 1, **caractérisé en ce que** tant les tubes (23, 231, 232) comme les barres (35, 1039, 1038, 1037, 1036) sont fixés aux points centraux sur aux deux des faces configurant le support interne.

5. L'implant selon la revendication 1, **caractérisé en ce que** tant les tubes (23, 231, 232) comme les barres (35, 1039, 1038, 1037, 1036) sont fixés à des points situés entre le centre et la surface postérieure seulement à deux côtés opposés du support interne.

6. L'implant selon la revendication 1, **caractérisé en ce que** le diamètre des barres est inférieur a 8 mm

7. L'implant selon la revendication 4, **caractérisé en ce que** el diamètre des barres est de 4 mm.

8. L'implant selon la revendication 1, **caractérisé en ce que** les barres sont moletés afin d'obtenir des sections d'un diamètre plus petit que le diamètre prédéterminé de la barre pour réaliser la coupure des barres.

9. L'implant selon la revendication 8, **caractérisé en ce que** le moletage de la barre est denté et **en ce que** le tube correspondant inclut un dispositif (703) pour entraver le coulissage de la barre dans le tube dans une direction, préférablement dans la direction correspondante au rapprochement des pièces de séparation vertébrale et ajustage télescopique

10. L'implant selon la revendication 16, **caractérise en ce qu'**il comporte quatre pièces :
une pièce d'appui supérieur (1) composé d'un membre horizontal (11) et d'une prolongation verticale (14) pour son ajustage à la face latérale vertébrale moyennant des vis;
une pièce d'ajustage télescopique (2) composée d'un membre plat (21) approprié pour son ajustage à la pièce de support supérieur et au moins deux prolongations verticales consistantes en des tubes (23, 231, 232) perpendiculaires par rapport au membre horizontal;
une pièce de séparation vertébrale (3) comportant un membre plat (31) et une quantité égale à celle de la pièce d'ajustage télescopique, de prolongations verticales consistantes en des barres (35) perpendiculaires par rapport au membre horizontal qui seront introduites dans les tubes de la pièce d'ajustage télescopique;
une pièce d'appui inférieur (4) comportant un membre horizontal (41) et une prolongation verticale (45) pour son ajustage à la face latérale vertébrale moyennant de vis.

11. L'implant selon la revendication 10, **caractérisé en ce que** les membres horizontaux (11, 41) des pièces d'appui supérieur e inférieur (1, 4), et les membres horizontaux de la pièce d'ajustage télescopique et la pièce de séparation vertébrale ont la forme qu'on choisit du groupe défini par les surfaces corticales des plateaux vertébraux des vertèbres lombaires en forme de bague vertébrale, des vertèbres cervicales en forme de bague configurée par deux lettres « E » vis-à-vis et des vertèbres thoraciques en forme de bague triangulaire.

12. L'implant selon la revendication 10, **caractérisé en ce que** la pièce d'appui inférieur (4) soit comporte un membre plat et une prolongation plate qui s'adapte au sacrum, le membre plat en forme de bague rectangulaire étant adaptable et ajustable à la pièce de séparation vertébrale.

13. L'implant selon la revendication 10, **caractérisé en ce que** les pièces d' appui vertébral supérieur et inférieur (1, 4) sont un membre horizontal en cale configurant un angle destiné au rétablissement de la courbature spinale.

14. L'implant selon la revendication 16, **caractérisé en ce que** l'union des pièces entre elles se fait manuellement moyennant des vis d'ajustage et d'attachement.

15. L'implant selon la revendication 1, **caractérisé en ce qu'**elle comporte deux pièces: une pièce d'appui supérieur et d'ajustage télescopique (1010, 310) comprenant un membre horizontal et une prolongation verticale pour son ajustage à la face latérale vertébrale moyennant des vis, et au moins deux prolongations verticales consistantes en des tubes perpendiculaires par rapport au membre horizontal; une pièce d'appui inférieur et de séparation vertébrale (1020, 320) composée d'un membre plat et d'une quantité égale a celle de la pièce d'ajustage télescopique, des prolongations verticales consistantes des barres perpendiculaires par rapport au membre horizontal qui seront introduites dans les tubes de la pièce d'ajustage télescopique, et une prolongation verticale pour son ajustage à la face latérale vertébrale moyennant des vis.

16. L'implant selon la revendication 15, **caractérisé en ce que** les membres horizontaux des pièces ont une forme choisie parmi le groupe défini par les surfaces corticales des plateaux vertébraux des vertèbres lombaires en forme de bague vertébrale, des vertèbres cervicales en forme de bague configurée par deux lettres "E" vis-à-vis et des vertèbres thoraciques en forme de bague triangulaire.

17. L'implant selon la revendication 15, **caractérisé en ce que** les membres horizontaux des pièces comportent un membre plat et une prolongation plate qui s'adapte au sacrum, en forme de bague rectangulaire.

18. L'implant selon la revendication 15, **caractérisé en ce que** les membres horizontaux des pièces sont constitués en forme de cale configurant un angle pour le rétablissement de la courbature spinale.

19. L'implant selon la revendication 10, **caractérisé en ce que** l'union entre les pièces d'appui et les pièces de séparation vertébrale et d'ajustage télescopique, présentent des mécanismes d'emboîtement réciproque.

20. L'implant selon la revendication 10 et 15 **caractérisé en ce que** les surfaces d'appui vertébral des pièces présentent des surfaces non lisses.

21. L'implant selon la revendication 20, **caractérisé en ce que** les surfaces d'appui vertébral des pièces présentent des surfaces rugueuses.

22. L'implant selon la revendication 20, **caractérisé en ce que** les surfaces d'appui vertébral des pièces présentent des surfaces dentées.

23. L'implant selon la revendication 10 et 15, **caractérisé en ce que** les prolongations latérales qui s'ajustent contre la face vertébral latérale permettent l'insertion d'une vis oblique par rapport à l'axe longitudinal des vertèbres.

24. L'implant selon la revendication 10 et 15, **caractérisé en ce que** la pièce d'appui vertébral et la pièce de séparation vertébrale comportent une unique pièce constituée de trois membres plats comprenant au moins des angles obtus entre eux, et leur membre inférieur inclut des barres verticales; son membre plat central inclut des orifices pour le passage des vis, cette configuration étant apte pour sa mise en place à l'axis.

25. L'implant selon la revendication 10 et 15, **caractérisé en ce que** les prolongations verticales des pièces d'appui vertébral incluent un système de butés (701, 702, 703) pour éviter le coulissement longitudinal des vis pénétrant dans le corps vertébral.

26. L'implant selon la revendication 25, **caractérisé en ce que** ledit système de butées (701, 702, 703) comporte une cannelure dans la prolongation latérale où se trouvent les orifices pour les vis pénétrant dans le corps vertébral, cette cannelure ayant une forme rectangulaire aces des extrémités supérieures et inférieures semi-circulaires; l'une des extrémités de la cannelure contiendra l'orifice pour le passage des vis; l'autre extrémité contiendra une butée coulissable à forme circulaire et une cannelure à façon de guide pour une saillie du type de barre à tête; ladite cannelure s'oriente verticalement pour permettre le coulissage vers la tête de la vis et continue ladite cannelure avec une prolongation dans le sens de la circonférence qui lui permet de tourner, en évitant de cette façon le coulissage de la butée.

27. L'implant selon la revendication 1, **caractérisé en ce qu'**elle comporte cinq pièces :
une pièce d'appui supérieur et une autre pièce d'appui inférieur comprenant un membre horizontal, que l'on fixe à une pièce de ajustage télescopique et de séparation vertébrale, indistinctement, moyennent des lames en saillies verticales qui s'emboîtent dans des cannelures latérales de la pièce d'ajustage télescopique ou de séparation vertébrale;
une pièce de prolongation verticale supérieure et une autre pièce inférieure pour son ajustage a la face latérale vertébrale moyennant des vis incluant des orifices pour ce but et qui s'ajuste à la pièce d'ajustage télescopique ou de séparation vertébrale moyennant deux barres horizontales qui sont insérés dans des orifices horizontaux correspondants dans la pièce d'ajustage télescopique ou de séparation vertébrale, dont l'attachement final est réalisé moyennant des vis qui son insérées dans des orifices situés aux faces latérales des pièces d'ajustage télescopique et de séparation vertébrale;
une pièce d'ajustage télescopique composée d'un membre plat approprié pour son ajustage à la pièce d'appui supérieur, que inclut des cannelures à au moins deux de ses faces latérales pour l'emboîtement dans les pièces d'appui vertébral, et au moins deux orifices qui se prolongent horizontalement pour contenir deux tubes de la pièce de prolongation verticale, et au moins deux prolongations verticales consistantes en des tubes perpendiculaires par rapport au membre horizontal;
une pièce de séparation vertébrale composé d'un membre plat et d'une quantité égale à celle de la pièce d'ajustage télescopique de prolongation verticale consistante en des barres perpendiculaires par rapport au membre horizontal que seront engagés dans les tubes de la pièce d'ajustage télescopique, la pièce de séparation vertébrale incluant des cannelures à au moins deux de ses faces latérales pour l'emboîtage dans les pièces d'appui vertébral, et au moins deux orifices qui se prolongent horizontalement pour contenir deux tubes de la pièce de prolongation verticale.

28. L'implant selon la revendication 1, 10, 15 et 27, **caractérisé en ce que** les barres et tubes sont fixés avec inclinaison par rapport aux pièces les assujettissant, permettant de pourvoir une courbature à l'ensemble de l'implant.

29. L'implant selon la revendication 1, 10, 15 et 27, **caractérisé en ce qu'**elle comporte des échancrures et des discontinuités en vue de l'utilisation d'une pince avec des butées permettant la séparation vertébrale et la mise en place de l'implant moyennant ledit instrument.

30. L'implant selon la revendication 2, **caractérisé en ce que** le patron amovible inclut à sa surface supérieure une série d'ailettes orientées offrant une résistance au coulissage.

31. L'implant selon la revendication 2, **caractérisé en ce que** la cale formant le patron peut avoir des angles de 0 degré, 1 degré, 2 degrés et 3 degrés.

32. L'implant selon la revendication 1, **caractérisé en ce que** les tubes et les barres se trouvent fixés aux sommets des supports internes.

33. L'implant selon la revendication 3, **caractérisé en ce que** l'inclinaison du membre horizontal de la pièce supérieure et inférieure par rapport au membres horizontaux, pour la correction angulaire de la colonne vertébrale, est choisie parmi les valeurs suivantes: 0 degré, 5 degrés et 9,5 degrés.

34. L'implant selon la revendication 1, **caractérisé en ce que** la prolongation verticale a une forme courbée pour ajuster le dispositif à la face latérale de la vertèbre.

35. L'implant de la revendication 1, **caractérisé en ce que** la prolongation verticale inclut des encoches horizontales à des distances prédéterminées.

36. L'implant de la reivendication1, **caractérisé en ce que** la pièce supérieure inclut un écran optionnel amovible, fixée moyennant une vis à la pièce à loger dans la surface antérieure afin d'éviter la migration d'os en dehors de la surface vertébrale.

37. L'implant des revendications 1, 10, 15 et 27, **caractérisé en ce qu'**il inclut seulement deux barres et deux tubes correspondants comme moyens de séparation.

38. L'implant selon la revendication 2, **caractérisé en ce que** le patron est fait de microgranules de titanium.

39. L'implant selon la revendication 1, 10, 15, et 27, **caractérisé en ce que** les barres et les tubes admettent un ajustage d' 11 mm.
